# EUROPÄISCHE PATENTSCHRIFT

(11) **EP 2 061 562 B1**
(45) Veröffentlichungstag und Bekanntmachung des Hinweises auf die Patenterteilung: **13.07.2016**
(21) Anmeldenummer: 07822562.0
(22) Anmeldetag: 13.11.2007
(51) Int. Cl.: A61Q 5/02, A61Q 5/10, A61Q 5/12, A61Q 19/00, A61Q 19/10, A61K 8/39, A61K 8/46, A61K 8/60, A61K 8/81, A61K 8/84

(54) **KONDITIONIERENDE ZUSAMMENSETZUNG VON BESONDERS AUSGEWÄHLTEN MILDEN ANIONISCHEN TENSIDEN UND KATIONISCHEN ODER AMPHOTEREN POLYMEREN IN MITTELN ZUR BEHANDLUNG KERATINISCHER FASERN**
CONDITIONING COMPOSITION OF SPECIALLY SELECTED MILD ANIONIC SURFACTANTS AND CATIONIC OR AMPHOTERIC POLYMERS IN AGENTS FOR THE TREATMENT OF KERATINIC FIBERS
COMPOSITION CONDITIONNANTE DE POLYMÈRES AMPHOTÈRES OU CATIONIQUES ET DE TENSIOACTIFS ANIONIQUES DOUX SPÉCIALEMENT SÉLECTIONNÉS DANS DES MOYENS DE TRAITEMENT DE FIBRES KÉRATINIQUES

(30) Priorität: 16.12.2006 DE 102006059569
(43) Veröffentlichungstag der Anmeldung: 27.05.2009
(73) Patentinhaber: Henkel AG & Co. KGaA, 40589 Düsseldorf (DE)
(72) Erfinder: HÖFFKES, Horst, 40595 Düsseldorf (DE); MAUSBERG, Sandra, 42781 Haan (DE); BROCKMANN, Claudia, 40627 Düsseldorf (DE); BOSSMANN, Britta, 40699 Erkrath (DE)
(86) Internationale Anmeldenummer: PCT/EP2007/062293
(87) Internationale Veröffentlichungsnummer: WO 2008/074576

(56) Entgegenhaltungen:
- EP-A- 0 392 320
- EP-A- 0 524 434
- EP-A- 0 531 650
- WO-A-95/15150
- WO-A-97/04743
- CA-A- 1 018 893
- US-A- 5 068 324
- US-A1- 2004 224 862
- US-A1- 2006 019 846
- US-B1- 6 290 944
- US-B1- 6 511 669
- US-B1- 6 514 488

## Beschreibung

"Konditionierende Zusammensetzung von besonders ausgewählten milden anionischen Tensiden und kationischen oder amphoteren Polymeren in Mitteln zur Behandlung keratinischer Fasern"

Die Erfindung betrifft eine konditionierende Zusammensetzung enthaltend besonders ausgewählter milder anionischer Tenside und kationischen oder amphoteren Polymeren in Mitteln zur Behandlung keratinischer Fasern.

Die kosmetische Behandlung von Haut und Haaren ist ein wichtiger Bestandteil der menschlichen Körperpflege. So wird menschliches Haar heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten.

Die bekannten Wirkstoffe können jedoch nicht alle Bedürfnisse in ausreichendem Maße abdecken. Es besteht daher weiterhin ein Bedarf nach Wirkstoffen bzw. Wirkstoffkombinationen für kosmetische Mittel mit guten pflegenden Eigenschaften und guter biologischer Abbaubarkeit. Insbesondere in farbstoff- und/oder elektrolythaltigen Formulierungen besteht Bedarf an zusätzlichen pflegenden Wirkstoffen, die sich problemlos in bekannte Formulierungen einarbeiten lassen.

Weiterhin ist die Verträglichkeit der kosmetischen Zusammensetzungen ein außerordentlich wichtiges Kriterium. In den einschlägigen dermatologischen Fachzeitschriften häufen sich die Berichte von zunehmenden Unverträglichkeiten in breiten Teilen der Bevölkerung auf alltägliche Produkte. Diese Unverträglichkeiten beruhen unter anderem auf den veränderten Verbrauchergewohnheiten und der Verfügbarkeit der exotischsten Rohstoffe und Lebensmittel im täglichen Umgang. Den Verbrauchern wird daher häufig geraten sich beispielsweise bei ihrer Ernährung einheimischer Produkte zu bedienen. Eine besonders große Herausforderung besteht daher für den Kosmetikchemiker darin, Kosmetika zur Reinigung und Pflege von Haut und Haar bevorzugt aus besonders verträglichen und bereits hervorragend bewährten Rohstoffen zu entwickeln. Ein besonderes Augenmerk gilt dabei besonders gezielt ausgewählten Rohstoffen, welche multifunktionale Aufgaben in der Rezeptur erfüllen können, damit möglichst wenige Inhaltsstoffe in einer Rezeptur enthalten sind. Je weniger Inhaltsstoffe in einer Rezeptur enthalten sind, je besser diese Inhaltsstoffe bekannt sind, desto geringer ist das Risiko einer Unverträglichkeit. Sollte es dennoch in Ausnahmefällen zu einer überempfindlichen Reaktion kommen, so kann der Allergologe aufgrund der wenigen Inhaltsstoffe sehr schnell den irritierenden Inhaltsstoff für diesen Verbraucher bestimmen. Die Kosmetika werden dadurch insgesamt sicherer in der Anwendung und in der Verträglichkeit für den Verbraucher.

Aufgabe der vorliegenden Erfindung ist daher die Bereitstellung von tensidischen Komponenten für Mittel zur Behandlung und Konditionierung keratinischer Fasern, insbesondere zur Reinigung und Pflege keratinischer Fasern, welche ganz besonders bevorzugt im Anschluß auf zuvor oxidativ behandelten keratinischen Fasern angewendet werden können. Gleichzeitig wird die Hautreizwirkung dieser Mittel vermindert. Im Anschluß an Mittel zur oxidativen Färbung von Haaren wird weiterhin vorzugsweise die Farbstabilität der Ausfärbung trotz des anschließenden Wasch- und Konditionierprozesses deutlich verbessert. In Mitteln zur Durchführung einer Kaltwelle werden bei der anschließenden Anwendung der erfindungsgemäßen Mittel das Volumen und die Haltbarkeit der Kaltwelle nicht nachteilig beeinflusst. Vielmehr verbleibt das Haar trotz einer hervorragenden Konditionierung haltbarer und mit mehr Volumen sowie ohne jegliche Belastung im Vergleich zu konventionellen Mitteln.

Die bekannten Zusammensetzungen lösen diese Aufgabe nicht. So offenbart die Schrift EP 0531650 A Haarwaschmittel enthaltend Natriumlaurylethersulfat und/oder Laurylethersulfosuccinat sowie kationische Cellulose. Die Schrift EP 0524434 A offenbart Haarwaschmittel enthaltend Fettsäureethercarbonsäuren und kationische Acrylat Copolymere. Die Schrift WO 97/04743 A offenbart Zusammensetzungen enthaltend Laurylethercarbonsäure und Natriumlaurylethersulfat sowie Polyquaternium-10. Die Schrift US 6511669 B offenbart Natriumlaurylethercarboxylat mit Merquat® 550. In der Patentschrift US 6514488 B wird Natriumlaurylethercarboxylat mit Vinylpyrrolidon/Methacrylamidopropyldimethylamin Copolymeren offenbart. In der Anmeldung US 2004/0224862 A wird ein Laurylglucosidcarboxylat mit einem kationischen Polymer offenbart. Schließlich offenbart die Patentschrift US 6290944 B eine Zusammensetzung aus Natriumlaurylethercarboxylat und einem kationischen Polymer. Es findet sich in keiner der genannten Offenbarungen des Standes der Technik ein Hinweis auf die vorliegende Erfindung.

Die Aufgaben werden erfindungsgemäß gelöst durch konditionierende Zusammensetzungen, welche als ersten zwingenden Bestandteil milde anionische Tenside enthalten, dadurch gekennzeichnet, dass als milde anionische Tenside eine Kombination aus a1) mindestens einer Ethercarbonsäure und a2) mindestens einem carboxilierten Alkylpolyglucosid verwendet wird.

Unter dem Begriff "milde Tenside" versteht der Fachmann Tenside, welche sich in den zahlreichen Testmethoden wie dem HET-CAM Test, dem Neutralrottest, dem BUS - Modell (bovine udder skin model), dem Humanhautmodell, dem Zeintest, dem Draize Test, dem Armflexwashtest oder dem Duhringkammertest usw. als milde Tenside erwiesen haben. Allen Testmodellen gemein ist, dass prinzipiell gegen einen Standard gemessen wird, auf welchen die Messergebnisse bezogen werden. In jedem dieser Testmodelle gibt es einen Schwellenwert unterhalb dessen von "milden Tensiden" gesprochen wird. Dieser Schwellenwert beträgt beispielsweie im HET-CAM-Test 1,5. Das bedeutet, dass als "mild" alle Tenside bezeichnet werden, die beispielsweise im HET-CAM-Test einen relativen Reizscore von 1,5 und kleiner aufweisen. Der Fachmann weiß, dass ein Tensid in jedem Testmodell einen anderen Score ergibt. Das bedeutet, dass beispielsweise ein Cocamidopropylbetain im HET-CAM-Test sogar als "reizend" eingestuft sein kann, während es in den anderen Testmodellen eher den milden Tensiden zugerechnet wird. Eine geläufige und anerkannte Einstufung definiert anionische Tenside als mild, wenn sie im HET-CAM-Test einen relativen Reizscore von kleiner als 1,5 aufweisen. Erfindungsgemäß bevorzugt werden jedoch solche anionischen Tenside als "milde anionische Tenside" verwendet und verstanden, welche in allen derzeit geläufigen Testmodellen als "mild" eingestuft werden.

Nach diesen Prüfmethoden haben sich die folgenden anionischen Tenside als mild bis besonders mild erwiesen:
Acyllactylate,
Hydroxymischethersulfate,
Ethercarbonsäuren der Formel R-O-(CH2-CH2O)x-CH2-COOH, in der R eine lineare Alkylgruppe mit 8 bis 30 C-Atomen und x = 0 oder 1 bis 16 ist und deren Salze,
Acylsarcoside mit 8 bis 24 C-Atomen in der Acylgruppe,
Acyltauride mit 8 bis 24 C-Atomen in der Acylgruppe,
Acylisethionate mit 8 bis 24 C-Atomen in der Acylgruppe, sind seit langem bekannte, hautfreundliche oberflächenaktive Stoffe, die durch Veresterung von Fettsäuren mit dem Natriumsalz der 2-Hydroxyethan-sulfonsäure (Isethionsäure) zugänglich sind. Wenn man für diese Veresterung Fettsäuren mit 8 bis 24 C-Atomen, also z. B. Laurin-, Myristin-, Palimitin- oder Stearinsäure oder auch technische Fettsäurefraktionen, z. B. die aus Kokosfettsäure erhältliche C₁₂ - C₁₈-Fettsäurefraktion einsetzt, erhält man die C₁₂ - C₁₈-Acylisethionate. Es ist bekannt, die Natriumsalze von C₁₂ - C₁₈-Acylisethionaten ähnlich wie Seifen auf Fettsäurebasis durch Kneten, Pilieren, Strangpressen, Extrudieren, Schneiden und Stückpressen in eine geeignete Form für den Transport und für die Anwendung zu bringen. Auf diese Weise lassen sich Nadeln, Granulate, Nudeln, Riegel und handliche Toilettenseifen-Stücke erzeugen.

Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen. Die Sulfobernsteinsäuremonoalkyl(C₈-C₂₄)-ester-dinatriumsalze werden nach bekanntem Verfahren z. B. dadurch hergestellt, daß man Maleinsäureanhydrid mit einem Fettalkohol mit 8 - 24 C-Atomen zum Maleinsäuremonoester des Fettalkohols umsetzt und diesen mit Natriumsulfit zum Sulfobernsteinsäureester sulfitiert. Besonders geeignete Sulfobernsteinsäureester leiten sich von Fettalkoholfraktionen mit 12 - 18 C-Atomen ab, wie sie z. B. aus Kokosfettsäure oder Kokosfettsäuremethylester durch Hydrierung zugänglich sind. Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,

Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen, Alkyl- und/oder Alkenyletherphosphate der Formel (E1-I), 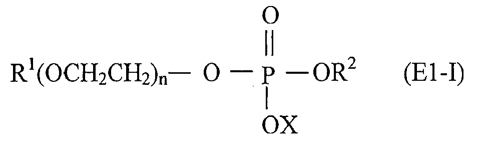 in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht, Glycerinethersulfate wie Monoglyceridsulfate und Monoglyceridethersulfate der Formel (E1-III) 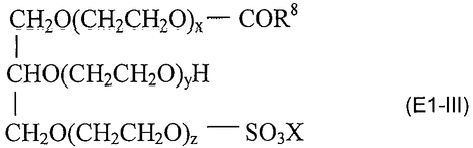 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für Mono-glycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäu-remonoglycerid und Talgfettsäuremonoglycerid sowie deren Ethylenoxidaddukte mit Schwefeltrioxid oder Chlorsulfonsäure in Form ihrer Natriumsalze. Geeignet sind Monoglyceridsulfate der Formel (E1-III) in der R⁸CO für einen linearen Acylrest mit 8 bis 18 Kohlenstoffatomen steht, Amidethercarbonsäuren, Kondensationsprodukte aus einem wasserlöslichen Salz eines wasserlöslichen Eiweißhydrolysat-Fettsäure-Kondensationsproduktes. Diese werden durch Kondensation von C8 - C30 Fettsäuren, bevorzugt von Fettsäuren mit 12 - 18 C-Atomen mit Aminosäuren, Mono-, Di- und wasserlöslichen Oligopeptiden und Gemischen solcher Produkte hergestellt, wie sie bei der Hydrolyse von Proteinen anfallen. Diese Eiweißhydrolysat-Fettsäure-Kondensationsprodukte werden mit einer Base neutralisiert und liegen dann bevorzugt als Alkali-, Ammonium-, Mono-, Di- oder Trialkanolammoniumsalz vor. Solche Produkte sind unter dem Warenzeichen Lamepon^{®}, Maypon^{®}, Gluadin^{®}, Hostapon^{®} KCG oder Amisoft^{®} seit langem im Handel erhältlich,
Acylglutamate und
Acylaspartate.

Sofern die milden anionischen Tenside Polyglycoletherketten enthalten, ist es ganz besonders bevorzugt, dass diese eine eingeengte Homologenverteilung aufweisen. Fettalkoholethersulfate mit einer eingeschränkten Homologenverteilung werden auch als "narrow range Fettalkoholethersulfate" bezeichnet. Weiterhin ist es im Falle von milden anionischen Tensiden mit Polyglycolethereinheiten bevorzugt, dass die Zahl der Glykolethergruppen 1 bis 20 beträgt, bevorzugt 2 bis 15, besonders bevorzugt 2 bis 12. Besonders milde anionische Tenside mit Polyglykolethergruppen ohne eingeschränkte Homologenverteilung können beispielsweise auch erhalten werden, wenn einerseits die Zahl der Polyglykolethergruppen 4 bis 12 beträgt und als Gegenion Zn- oder Mg-ionen gewählt werden. Beispiele hierfür sind das Handelsprokt Texapon^{®} ASV.

Selbstverständlich können alle bislang und im folgenden genannten milden anionischen Tenside auch in Form ihrer Salze verwendet werden. Besonders geeignete milde anionische Tenside liegen jeweils in Form der Lithium-, Magnesium-, Zink-, Natrium-, Kalium- und Ammonium- sowie der Mono-, Di- und Trialkanolammoniumsalze mit 1 bis 4 C-Atomen in der Alkanolgruppe vor. Die bevorzugten Ammoniumionen sind neben dem Ammoniumion als solchem Monomethylammonium-Dimethylammonium-, Trimethylammonium-, Monoethylammonium-, Diethylammonium-, Triethylammonium-, Monopropylammonium-, Dipropylammonium-, Tripropylammonium-, Monoisopropylammonium-, Diisopropylammonium-, Triisopropylammonium-, Monobutylammonium-, Dibutylammonium-, Tributylammonium-, Monoisobutylammonium-, Diisobutylammonium-, Triisobutylammonium-, Mono-t-butyl-ammonium-, Di-t-butyl-ammonium-, Tri-t-butyl-ammoniumionen sowie gemischte Ammoniumionen wie beispielsweise Methyl-ethyl-ammonium-, Dimethyl-ethyl-ammonium-, Methyl-diethylammonium-, Methyl-propyl-ammonium-, Methyl-ethyl-propyl-ammonium-, Ethyl-diisopropylammonium-, Ethyl-dibutyl-ammonium-, Ethyldiisobutylammoniumionen usw. Selbstverständlich umfasst die erfindungsgemäße Lehre auch die weiteren nicht explizit genannten Ammoniumionen dieser Alkanolammoniumsalze.

Weitere milde anionische Tenside sind Alkyl- und/oder Alkenyl-Oligoglykosidcarboxylate, - sulfate, -phosphate und/oder -isethionate, die sich von Alkyl- und/oder Alkenyloligoglykosiden der allgemeinen Formel (I) ableiten,

R-O-(G)ₚ (I)

mit der Bedeutung
- R: C₆₋₂₂-Alkyl oder C₆₋₂₂-Alkenyl,
- G: Glykosideinheit, die sich von einem Zucker mit 5 oder 6 Kohlenstoffatomen ableitet,
- p: Zahl von 1 bis 10.

Erfindungsgemäß ist die Verwendung einer Kombination aus mindestens einer Ethercarbonsäure und mindestens einem carboxilierten Alkylpolyglucosid.

Das Mischungsverhältnis dieser Tenside untereinander beträgt mindestens 10:1 bis 1:10. Bevorzugt ist ein Mischungsverhältnis von 5 : 1 bis 1 : 5, besonders bevorzugt von 2,5 : 1 bis 1 : 2,5 und am bevorzugtesten von etwa 1,5:1 bis 1 : 1,5. Es wurde erfindungsgemäß gefunden, dass die Verwendung von milden anionischen Tensiden in Mitteln zur Behandlung keratinischer Fasern, insbesondere in Mitteln zur Haarreinigung und -konditionierung zu einer Verminderung der Hautreizung führt. Ferner wurde gefunden, dass bei der Anwendung derartiger Mittel die Farbstabilität der Ausfärbung durch Mitverwendung der milden anionischen Tenside verbessert wird. Insbesondere wird diese Wirkung bei Anwendung auf strapaziertem Haar erreicht. Auch die Waschechtheit von gefärbtem strapaziertem Haar wird verbessert.

Darüber hinaus wurde erfindungsgemäß gefunden, dass bei Verwendung milder anionischer Tenside in Mitteln zur Reinigung und Pflege bei der Anwendung dieser Mittel das Aufschäumen erheblich verbessert wird. Der Schaum zeichnet sich insbesondere durch ein feinporiges, dichtes, cremiges Erscheinungsbild aus. Der Schaum wird als angenehm weich und geschmeidig und leicht verteilbar beschrieben. Gleichzeitig ist der Schaum fest und gut greifbar. Er zeigt ein gewisses Standvermögen und verläuft nicht spontan sondern erst nach einigen Minuten. Dies begünstigt die bereits beschriebene leichte Verteilbarkeit des Schaumes. Diese Effekte treten in den erfindungsgemäßen Zusammensetzungen insbesondere durch die Kombination mit kationischen und / oder amphoteren Polymeren auf.

Die milden anionischen Tenside zeigen eine synergistische Wirkung mit den kationischen und/oder amphoteren Polymeren. Diese synergistische Wirkung beruht möglicherweise auf einer verstärkten Abscheidung dieser Polymere auf der Oberfläche von Haut und Haar, was sich im gesamten kosmetischen Erscheinungsbild von Haut und Haar bemerkbar macht. Die besonderen Ladungseffekte von kationischen und amphoteren Polymeren scheinen hierbei eine Rolle zu spielen. Das Haar wird beispielsweise sowohl im trockenen wie auch im nassen Zustand besser kämmbar, leichter frisierbar und zeigt mehr Glanz sowie einen angenehmeren Griff.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern verwendet werden. In den Alkyl- und/oder Alkenyl-Oligoglykosiden ist vorzugsweise in mindestens einem der Reste G mindestens eine Hydroxylgruppe durch -O-C₁₋₁₂-Alkenyl-COOM, -OSO₃M, -OP(O)(OM)₂ oder-O-CH₂-CH₂-SO₃M mit M = H, Alkalimetall, NH₄ oder einem der bereits zuvor genannten Gegenionen wie Zn, Mg, Alkanolammonium ersetzt.

Dabei wird besonders bevorzugt ein Alkyloligoglykosid-Carboxylat eingesetzt, in dem -O-C₁₋₁₂-Alkylen-COOM -O(CH₂-)ₙCOOM mit M = H, Na oder K und n = 1 bis 3 bedeutet. Besonders bevorzugt ist der Rest O-CH₂-COONa.

Besonders bevorzugt wird ein Alkyloligoglykosidcarboxylat eingesetzt, in dem der Alkylrest ein Laurylrest ist. Speziell bevorzugt ist ein Laurylglucosidcarboxylat, wie es als Plantapon^{®} LCG von Cognis Deutschland erhältlich ist.

Die Indexzahl p in der allgemeinen Formel (I) gibt den Oligomerisierungsgrad, d.h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkylglykosid eine analytische ermittelte rechnerische Größe, die meistens eine gebrochene Zahl darstellt. Vorzugsweise werden Alkylglykoside mit einem mittleren Oligomerisierungsgrad p von 1,1 bis 3,0 verwendet. Besonders bevorzugt sind solche Alkylglykoside, deren Oligomerisierungsgrad kleiner als 1,5 ist und insbesondere zwischen 1,1, und 1,4 liegt.

Der Alkylrest R leitet sich von primären Alkoholen mit 6 bis 22, vorzugsweise 12 bis 18 Kohlenstoffatomen ab. Typische Beispiele sind Capronalkohol, Caprylalkohol, Caprinalkohol, Laurylalkohol, Myristylalkohol, Cetylalkohol, Stearylalkohol und Behenylakohol sowie technische Fraktionen, die neben den genannten gesättigten Alkoholen auch Anteile an ungesättigten Alkoholen enthalten können und die auf Basis von natürlichen Fetten und Ölen, beispielsweise Palmöl, Palmkernöl, Kokosöl oder Rindertalg gewonnen werden. Der Einsatz von technischem Kokosalkohol ist hierbei besonders bevorzugt.

Neben den genannten Fettalkoholen können sich die Alkylglykoside auch von synthetischen primären Alkoholen mit 6 bis 22 Kohlenstoffatomen, insbesondere den sogenannten Oxoalkoholen ableiten, die einen Anteil von 5 bis 40 Gew.-% verzweigter Isomeren aufweisen.

Besonders bevorzugte Alkylreste sind solche mit 8/10, 12/14, 8 bis 16, 12 bis 16 oder 16 bis 18 C-Atomen. Mischungen der Alkylreste ergeben sich bei einer Herstellung ausgehend von natürlichen Fetten und Ölen oder Mineralölen.

Verfahren zur Herstellung dieser Alkylglykoside sind dem Fachmann seit langem bekannt

Die Herstellung der Alkyl- oder Alkenyl-Oligoglykosidcarboxylate, - phosphate, -sulfate oder -isethionate kann nach bekannten Verfahren erfolgen. Die Herstellung der Carboxylate erfolgt beispielsweise durch Umsetzung der Alkyloligoglykoside mit Salzen von Chlorcarbonsäuren in Gegenwart von Basen. Beispielsweise kann mit 2-Chloressigsäure-Natriumsalz in Gegenwart von NaOH umgesetzt werden. Bei der Umsetzung können sowohl die Hydroxylgruppen im Ring wie auch die -CH₂-OH-Gruppe umgesetzt werden. Der Umsetzungsgrad ist u.a. abhängig von der Stöchiometrie der Einsatzprodukte. Vorzugsweise werden die Alkyloligoglykoside zumindest an der -CH₂-OH-Gruppe umgesetzt, wobei optional ein Mittel eine oder mehreren der am Ring befindlichen Hydroxylgruppen umgesetzt werden können.

Weitere Hydroxylgruppen können beispielsweise auch verethert sein.

Die Herstellung der Isethionate erfolgt ebenfalls nach bekannten Verfahren des Standes der Technik. Ferner ist bekannt, dass die Produkte zur Haar- und Körperpflege eingesetzt werden können. Es werden insbesondere wässrige Detergenzgemische beschrieben, die Alkyloligoglykosidisethionate und beispielsweise weitere anionische Tenside enthalten.

Die Herstellung der Sulfate erfolgt ebenfalls nach bekannten Verfahren. Weiterhin sind Gemische der APG-Sulfate mit u.a. Alkylsulfaten oder Alkylethersulfaten sowie weiteren Inhaltsstoffen beschrieben. Es ist angegeben, dass die Tensidmischungen in Produkten eingesetzt werden können, die zum Waschen, Färben, Wellen oder zur Spülung von Haaren dienen.

Die Herstellung der Sulfate erfolgt ebenfalls gemäß dem Stand der Technik. Beispielsweise kann das entsprechende Alkylglykosid mit gasförmigem Schwefeltrioxid oder mit Schwefelsäure, gefolgt von Neutralisierung, umgesetzt werden. Kosmetische und pharmazeutische Zubereitungen enthaltend die Alkyloligoglykosidsulfate sind ebenfalls bekannt.

Schließlich sind Detergenzgemische aus Alkyloligoglykosidsulfaten und Alkyletherphosphaten beschrieben, die beispielsweise in Haarspülungen, Haarfärbemitteln oder Haarwellmitteln eingesetzt werden können.

Die erfindungsgemäß eingesetzten milden anionischen Tenside werden in einer Menge von 0,1 bis 25 Gew.-%, besonders bevorzugt 0,1 bis 15 Gew.-% und ganz besonders bevorzugt in einer Menge von 0,5 bis 10,0 Gew.% verwendet

Die eingesetzten milden anionischen Tenside können in diesen Mitteln die üblichen anionischen Tenside ganz oder teilweise ersetzen. Damit können die erfindungsgemäßen milden anionischen Tenside als alleiniges anionisches Tensid in den Mitteln eingesetzt werden, oder es können Gemische dieser milden anionischen Tenside untereinander oder mit üblichen weiteren anionischen Tensiden eingesetzt werden. Diese üblichen anionischen Tenside sind an späterer Stelle näher erläutert. Beispielsweise können die milden anionischen Tenside und weitere anionische Tenside in einem Gewichtsverhältnis im Bereich von 5 : 0,05 bis 1 : 2, besonders bevorzugt 3 : 0,5 bis 1 : 2, insbesondere 2,5 : 0,5 bis 1 : 1,5 und am bevorzugtesten 1,5 : 1 bis 1 : 1,5 vorliegen.

In den erfindungsgemäßen Zusammensetzungen sind weiterhin zwingend kationische und/oder amphotere Polymere enthalten. Diese beiden Gruppen von Polymeren haben eine potentiell kationische Ladung gemeinsam. Sowohl kationische als auch amphotere bzw. zwitterionische Polymere können daher über ihre kationische Ladungsdichte charakterisiert werden. Die erfindungsgemäßen Polymere zeichnen sich durch eine Ladungsdichte von mindestens 1 bis 7 meq/g aus. Eine Ladungsdichte von mindestens 2 bis 7 meq/g ist dabei bevorzugt. Besonders bevorzugt ist eine Ladungsdichte von mindestens gleich 3 meq/g bis 7 meq/g.

Ein weiteres charakteristisches Merkmal der erfindungsgemäßen Polymeren ist ihre Molmasse. Unter der Molmasse des jeweiligen Polymeren wird die Molmasse verstanden, welche der Hersteller in den entsprechenden Datenblättern nach dessen Methode gemessen angibt. Für die Auswahl eines geeigneten Polymeren hat sich eine Molmasse von mindestens 50.000 g/u als erfindungsgemäß geeignet erwiesen. Polymere mit einer Molmasse von mehr als 100.000 g/u haben sich als besonders geeignet erwiesen. Polymere mit einer Molmasse von mehr als 1000.000 g/u sind ganz besonders geeignet.

Definiert man die Adsorptionswahrscheinlichkeit als das Produkt aus der kationischen Ladungsdichte und der Molmasse, so kann dieses Produkt zur gezielten Auswahl geeigneter erfinderischer Polymere herangezogen werden.

Geeignete Polymere weisen für das Produkt aus der kationischen Ladungsdichte und der Molmasse einen Wert von größer als 100.000 auf. Besonders geeignet sind Polymere, welche für dieses Produkt einen Wert von mindestens 200.000 aufweisen. Ganz besonders geeignet sind diejenigen Polymere, bei welchen dieses Produkt einen Wert größer als 250.000 aufweist. Am geeignetsten sind diejenigen Polymere, bei welchen dieses Produkt einen Wert von mindestens 1.000.000 aufweist.

Unter kationischen Polymeren sind Polymere zu verstehen, welche in der Haupt- und/oder Seitenkette eine Gruppe aufweisen, welche "temporär" oder "permanent" kationisch sein kann.

Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Die erfindungsgemäßen kationischen Polymere können sowohl festigende und/oder filmbildende und/oder antistatische und/oder avivierende Polymere als auch Polymere mit konditionierenden und/oder verdickenden Eigenschaften sein. Bei den geeigneten kationaktiven Polymeren handelt es sich vorzugsweise um haarfestigende und/oder um haarkonditionierende Polymere. Unter Polymeren sind sowohl natürliche als auch synthetische Polymere, welche kationisch oder amphoter geladen sein können, zu verstehen.

Bevorzugt sind solche Polymere, die eine ausreichende Löslichkeit in Wasser oder Alkohol besitzen, um in dem erfindungsgemäßen Mittel bei der Anwendung auf dem feuchten bis nassen Haar vollständig in Lösung zu gehen.

Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium- 11) sowie quaternäre Silikonpolymere bzw. -oligomere wie beispielsweise Silikonpolymere mit quaternären Endgruppen (Quaternium-80).

Von den kationischen Polymeren, die in dem erfindungsgemäßen Mittel enthalten sein können, ist zum Beispiel Vinylpyrrolidon/Dimethylaminoethylmethacrylatmethosulfat Copolymer, das unter den Handelsbezeichnungen Gafquat^{®} 755 N und Gafquat^{®} 734 von der Firma Gaf Co., USA vertrieben wird und von denen das Gafquat^{®} 734 besonders bevorzugt ist, geeignet. Weitere kationische Polymere sind beispielsweise das von der Firma BASF, Deutschland unter dem Handelsnamen Luviquat^{®} HM 550 vertriebene Copolymer aus Polyvinylpyrrolidon und Imidazoliminmethochlorid, das von der Firma Calgon/USA unter dem Handelsnamen Merquat® Plus 3300 vertriebene Terpolymer aus Dimethyldiallylammoniumchlorid, Natriumacrylat und Acrylamid und das von der Firma ISP unter dem Handelsnamen Gafquat^{®} HS 100 vertriebene Vinylpyrrolidon/Methacrylamidopropyltrimethylammoniumchlorid Copolymer.

Ein besonders geeignetes Homopolymer ist das, gewünschtenfalls vernetzte, Poly(methacryloyloxyethyltrimethylammoniumchlorid) mit der INCl-Bezeichnung Polyquaternium-37. Solche Produkte sind beispielsweise unter den Bezeichnungen Rheocare^{®} CTH (Cosmetic Rheologies) und Synthalen^{®} CR (3V Sigma) im Handel erhältlich. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nicht wäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCl-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Geeignete Silikon polymere mit Aminogruppen sind unter der INCI- Bezeichnung Amodimethicone bekannt. Hierbei handelt es sich um Polydimethylsiloxane mit Aminoalkylgruppen. Die Aminoalkylgruppen können seiten- oder endständig sein. Das N-haltige Silikon als erfindungsgemäßes kationisches Polymer kann vorzugsweise ausgewählt werden aus der Gruppe umfassend Siloxanpolymere mit wenigstens einer Aminogruppe, Siloxanpolymere mit wenigstens einer endständigen Aminogruppe, Amodimethicon, Trimethylsilylamodimethicone, und/oder Aminoethylaminopropylsiloxan-Dimethylsiloxan-Copolymer. Geeignete Silikon polymere mit zwei endständigen quaternären Ammoniumgruppen sind unter der INCI-Bezeichnung Quaternium-80 bekannt. Hierbei handelt es sich um Dimethylsiloxane mit zwei endständigen Aminoalkylgruppen.

Erfindungsgemäß bevorzugt ist die Verwendung eines Aminosiloxans entsprechend der nachstehenden allgemeinen Formel (G1-II), 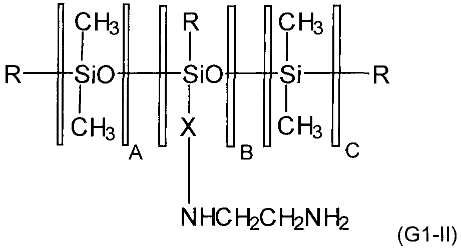 wobei R = OH oder CH₃ ; X = Alkylgruppe mit 1 bis 4 C-Atomen, vorzugsweise Propyl oder Isopropyl und A, B und C = Copolymereinheiten, die taktische und/oder ataktische Polymerblöcke ausbilden können, sind.

Erfindungsgemäß am meisten bevorzugt ist Amodimethicon, Amodimethicon haltige Emulsionen oder Fluide. Emulsionen, die erfindungsgemäß bevorzugt eingesetzt werden können sind Dow Corning® 949, hierbei handelt es sich um eine kationische Emulsion enthaltend Amodimethicon, Cetrimoniumchlorid und Trideceth-12; Dow Corning® 939, hierbei handelt es sich um eine Emulsion enthaltend Amodimethicon, Cetrimoniumchlorid und Trideceth-12; Dow Corning® 929, hierbei handelt es sich um eine kationische Emulsion enthaltend Amodimethicon, Talktrimoniumchlorid und Nonoxynol-10; Dow Corning® 7224 oder 1401, basierend auf Trimethylsilylamodimethicon, Octoxynol-40, Isolaureth-6 und Glycol; Dow Corning® 2-8194 Mikroemulsion (26%ig) auf Basis eines aminfunktionalisierten Siliconpolymers; Dow Corning® 2-8177 Mikroemulsion (12%ig) auf Basis eines aminfunktionalisierten Siliconpolymers; Dow Corning® 2-8566 Amino Fluid auf Basis eines aminfunktionalisierten Polydimethylsiloxans; erhältlich bei der Firma Dow Corning.

Das Silikon als erfindungsgemäßes kationisches Polymer wird in einer Menge von 0,01 bis 20 Gew.% bezogen auf das gesamte Mittel, bevorzugt in Mengen von 0,05 bis 15 Gew.% und ganz besonders bevorzugt in Mengen von 0,05 bis 10 Gew.% verwendet.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel (G1-III) G-O-B-N+RₐR_{b}R_{c} X⁻
G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
Rₐ, R_{b} und R_{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in Rₐ, R_{b} und R_{c} vorzugsweise maximal 20 ist;
X⁻ ist ein übliches Gegenanion und ist vorzugsweise Chlorid.

Eine kationische Cellulose wird unter der Bezeichnung Polymer JR^{®} von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Ein geeignetes kationisches Guarderivat wird unter der Handelsbezeichnung Jaguar^{®} vertrieben und hat die INCl-Bezeichnung Guar Hydroxypropyltrimonium Chloride.

Besonders bevorzugte kationaktive Stoffe sind Chitosan, Chitosansalze und Chitosan-Derivate. Ein bevorzugtes Chitosansalz ist Chitosoniumpyrrolidoncarboxylat, welches beispielsweise unter der Bezeichnung Kytamer^{®} PC von der Firma Amerchol, USA, vertrieben wird. Weitere Chitosanderivate sind unter den Handelsbezeichnungen Hydagen^{®} CMF, Hydagen^{®} HCMF und Chitolam^{®} NB/101 im Handel frei verfügbar.

Weitere bevorzugte kationische Polymere sind beispielsweise quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
kationische Alkylpolyglycoside gemäß der DE-PS 44 13 686,
kationiserter Honig, beispielsweise das Handelsprodukt Honeyquat^{®} 50,
kationische Guar-Derivate, wie insbesondere die unter den Handelsnamen Cosmedia^{®}Guar und Jaguar^{®} vertriebenen Produkte,
polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
Copolymere des Vinylpyrrolidons mit quaternierten Derivaten des Dialkylaminoalkylacrylats und - methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
Vinylpyrrolidon-Vinylimidazoliummethochlorid-Copolymere, wie sie unter den Bezeichnungen Luviquat^{®} FC 370, FC 550, FC 905 und HM 552 angeboten werden,
quaternierter Polyvinylalkohol,
sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette, Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Die kationischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Weiterhin können als Polymere amphotere Polymere (G3) verwendet werden. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO-oder -SO₃-Gruppen enthalten, und solche Polymere zusammengefaßt, die -COOH- oder SO₃H-Gruppen und quartäre Ammoniumgruppen enthalten.

Amphotere Polymere sind ebenso wie die kationischen Polymere ganz besonders bevorzugte Polymere.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Bevorzugt eingesetzte amphotere Polymere sind solche Polymerisate, die sich im wesentlichen zusammensetzen aus
(a) Monomeren mit quartären Ammoniumgruppen der allgemeinen Formel (G3-I),

   R¹-CH=CR²-CO-Z-(CₙH₂ₙ)-N⁽⁺⁾R³R⁴R⁵ A⁽⁻⁾ (G3-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (G3-II),

   R⁶-CH=CR⁷-COOH (G3-II)
in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Geeignete Ausgangsmonomere sind z. B. Dimethylaminoethylacrylamid, Dimethylaminoethylmethacrylamid, Dimethylaminopropylacrylamid, Dimethylaminopropylmethacrylamid und Diethylaminoethylacrylamid, wenn Z eine NH-Gruppe bedeutet oder Dimethylaminoethylacrylat, Dimethylaminoethylmethacrylat und Diethylaminoethylacrylat, wenn Z ein Sauerstoffatom ist.

Die eine tertiäre Aminogruppe enthaltenden Monomeren werden dann in bekannter Weise quarterniert.

Das Acrylamidopropyl-trimethylammoniumchlorid ist ein ganz besonders bevorzugtes Monomer der Formel (G3-I).

Als monomere Carbonsäuren der Formel (G3-II) eignen sich Acrylsäure, Methacrylsäure, Crotonsäure und 2-Methyl-crotonsäure. Bevorzugt werden Acryl- oder Methacrylsäure, insbesondere Acrylsäure, eingesetzt.

Die erfindungsgemäß einsetzbaren zwitterionischen Polymerisate werden aus Monomeren der Formeln (G3-I) und (G3-II) nach an sich bekannten Polymerisationsverfahren hergestellt.

Die amphoteren Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen. Weiterhin geeignete zwitterionische Polymere sind Methacroylethylbetain/Methacrylat-Copolymere, die unter der Bezeichnung Amersette^{®} (AMERCHOL) im Handel erhältlich sind.

Erfindungsgemäß ganz besonders bevorzugte kationische und/oder amphotere Polymere sind die Produkte mit den Handelsbezeichnungen Luviquat Style, Luviquat Excellence, Luviquat FC 550, Luviquat Care, Luviquat FC 370, sowie generell die Produkte der Luviquat - Serie, Polyethylenimine, Hexamethidrine, Polymere aus Piperazinyleinheiten, wasserlösliche Polyaminoamide, wie beispielsweise Cartaretine, Polyalkylenpolyamine wie beispielsweise Hercosett- oder Delsetttypen, Gafquat - Typen, Jaguar -Typen, Cosmedia Guar - Typen, Mirapol - Typen, Merquat - Typen oder das Polymer W 37194.

Typische Beispiele für anionische Tenside sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Beispiele für besonders geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
lineare und verzweigte Fettsäuren mit 8 bis 30 C-Atomen (Seifen),
lineare Alkansulfonate mit 8 bis 24 C-Atomen,
lineare Alpha-Olefinsulfonate mit 8 bis 24 C-Atomen,
Alpha-Sulfofettsäuremethylester von Fettsäuren mit 8 bis 30 C-Atomen, Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
Gemische oberflächenaktiver Hydroxysulfonate gemäß DE-A-37 25 030,
sulfatierte Hydroxyalkylpolyethylen- und/oder Hydroxyalkylenpropylenglykolether gemäß DE-A-37 23 354,
Sulfonate ungesättigter Fettsäuren mit 8 bis 24 C-Atomen und 1 bis 6 Doppelbindungen gemäß DE-A-39 26 344,
sulfatierte Fettsäurealkylenglykolester der Formel (E1-II)

R⁷CO(AlkO)ₙSO₃M (E1-II)

in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside (E2) werden solche oberflächenaktiven Verbindungen bezeichnet, die im Molekül mindestens eine quartäre Ammoniumgruppe und mindestens eine -COO⁽⁻⁾ - oder-SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCl-Bezeichnung Cocamidopropyl Betaine bekannte Fettsäureamid-Derivat.

Unter ampholytischen Tensiden (E3) werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine.

Besonders bevorzugte ampholytische Tenside sind das N-Kokosalkylaminopropionat, das Kokosacylaminoethylaminopropionat und das C₁₂- C₁₈ - Acylsarcosin.

Nichtionische Tenside (E4) sind beispielsweise

Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,

Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,

Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
C₁₂-C₃₀-Fettsäuremono- und -diester von Anlagerungsprodukten von 1 bis 30 Mol Ethylenoxid an Glycerin,
Anlagerungsprodukte von 5 bis 60 Mol Ethylenoxid an Rizinusöl und gehärtetes Rizinusöl,
Polyolfettsäureester, wie beispielsweise das Handelsprodukt Hydagen^{®} HSP (Cognis) oder Sovermol^{®} - Typen (Cognis),
alkoxilierte Triglyceride,
alkoxilierte Fettsäurealkylester der Formel (E4-I)

R¹CO-(OCH₂CHR²)_{w}OR³ (E4-I)

in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
Aminoxide,
Hydroxymischether, wie sie beispielsweise in der DE-OS 19738866 beschrieben sind,
Sorbitanfettsäureester und Anlagerungeprodukte von Ethylenoxid an Sorbitanfettsäureester wie beispielsweise die Polysorbate,
Zuckerfettsäureester und Anlagerungsprodukte von Ethylenoxid an Zuckerfettsäureester,
Anlagerungsprodukte von Ethylenoxid an Fettsäurealkanolamide und Fettamine,
Zuckertenside vom Typ der Alkyl- und Alkenyloligoglykoside gemäß Formel (E4-II),

R⁴O-[G]ₚ (E4-II)

in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Bevorzugt sind Alkyloligoglucoside auf Basis von gehärtetem C_{12/14}-Kokosalkohol mit einem DP von 1 bis 3.

Zuckertenside vom Typ der Fettsäure-N-alkylpolyhydroxyalkylamide, ein nichtionisches Tensid der Formel (E4-III), 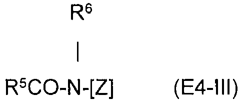 in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Die bevorzugten Fettsäure-N-alkylpolyhydroxyalkylamide stellen daher Fettsäure-N-alkylglucamide dar, wie sie durch die Formel (E4-IV) wiedergegeben werden:

R⁷CO-(NR⁸) -CH₂ - [CH(OH)]₄ - CH₂OH (E4-IV)

Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden.

Die Zuckertenside können in den erfindungsgemäß verwendeten Mitteln bevorzugt in Mengen von 0,1 - 20 Gew.-%, bezogen auf das gesamte Mittel, enthalten sein. Mengen von 0,5 - 15 Gew.-% sind bevorzugt, und ganz besonders bevorzugt sind Mengen von 0,5 - 7,5 Gew.%.

Weitere typische Beispiele für nichtionische Tenside sind Fettsäureamidpolyglycolether, Fettaminpolyglycolether, Mischether bzw. Mischformale, Proteinhydrolysate (insbesondere pflanzliche Produkte auf Weizenbasis) und Polysorbate.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure sowie die Zuckertenside erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Als Zusätze zur weiteren Verbesserung der Cremigkeit des Schaumes und des Hautgefühls während und nach der Anwendung haben sich auch nichtionische Tenside bewährt, deren zusätzliche Verwendung zur Herstellung der erfindungsgemäßen Zusammensetzungen empfohlen werden kann: Besonders bevorzugt sind daher erfindungsgemäße Zusammensetzungen mit einem zusätzlichen Gehalt von 0,1 - 20 Gew.-% an nichtionischen Tensiden mit einem HLB-Wert von 2 - 18.

Die kationischen Tenside (E5) bilden die letzte Gruppe von Tensiden. Kationische Tenside zeichnen sich als Teil des erfindungsgemäßen Wirkstoffkomplexes dadurch aus, dass sie wie die amphoteren und zwitterionischen Tenside zu einem deutlich verbesserten kosmetischen Erscheinungsbild von Haut und Haar beitragen. Die kationische Ladung sorgt für eine gute Bindung an den eher negativ geladenen Oberflächen insbesondere von geschädigtem Haar oder beanspruchter Haut. An den langen Fettresten dieser Molekülstrukturen wiederum können sich verstärkt eher hydrophob aufgebaute Wirkstoffe anlagern. Dadurch wird insgesamt eine erhöhte Abscheidung von Pflegestoffen auf der Oberfläche von Haut und Haar bewirkt. Das Haar wird beispielsweise sowohl im trockenen wie auch im nassen Zustand besser kämmbar, leichter frisierbar und zeigt mehr Glanz sowie einen angenehmeren Griff.

Kationische Tenside (E5) leiten sich im allgemeinen von Ammoniumionen ab und besitzen eine Struktur (NR¹R²R³R⁴)⁺ mit einem entsprechend negatv geladenen Gegenion. Derartige kationische Ammoniumverbindungen sind dem Fachmann bestens bekannt. Weitere kationische Tenside sind beispielsweise die Esterquats oder die Imidazoliumverbindungen. Erfindungsgemäß besonders bevorzugt einsetzbar sind kationische Tenside (E5) vom Typ der quarternären Ammoniumverbindungen, der Esterquats, der Imidazoline und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 8 bis 30 Kohlenstoffatome auf. Typische Beispiele für kationische Tenside sind quartäre Ammoniumverbindungen und Esterquats, insbesondere quaternierte Fettsäuretrialkanolaminester-Salze.

Besonders bevorzugt einsetzbar können erfindungsgemäß kationische Verbindungen mit Behenylresten, insbesondere die unter der Bezeichnung Behentrimoniumchlorid bzw. -bromid (Docosanyltrimethylammonium Chlorid bzw. -Bromid) bekannten Substanzen. Andere bevorzugte QAV weisen mindestens zwei Behenylreste auf. Kommerziell erhältlich sind diese Substanzen beispielsweise unter der Bezeichnungen Genamin^{®} KDMP (Clariant).

Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Als weitere kationische Tenside können die erfindungsgemäßen Mittel mindestens eine quartäre Imidazolinverbindung enthalten. Die im Folgenden dargestellte Formel (E5-V) zeigt die Struktur dieser Verbindungen.

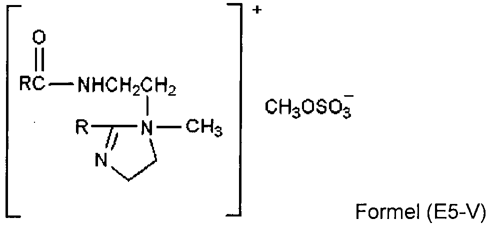

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (E5-V) enthalten für R jeweils den gleichen Kohlenwasserstoffrest. Die Kettenlänge der Reste R ist bevorzugt 12 Kohlenstoffatome. Besonders bevorzugt sind Verbindungen mit einer Kettenlänge von mindestens 16 Kohlenstoffatomen und ganz besonders bevorzugt mit mindestens 20 Kohlenstoffatomen. Eine ganz besonders bevorzugte Verbindung der Formel I weist eine Kettenlänge von 21 Kohlenstoffatomen auf. Ein Handelsprodukt dieser Kettenlänge ist beispielsweise unter der Bezeichnung Quaternium-91 bekannt. In der Formel (E5-V) ist als Gegenion Methosulfat dargestellt. Erfindungsgemäß umfasst sind jedoch als Gegenionen auch die Halogenide wie Chlorid, Fluorid, Bromid, oder auch Phosphate.

Die Imidazoline der Formel (E5-V) sind in den erfindungsgemäßen Zusammensetzungen in Mengen von 0,01 bis 20 Gew.%, bevorzugt in Mengen von 0,05 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar. Die Alkylamidoamine können sowohl als solche vorliegen und durch Protonierung in entsprechend saurer Lösung in eine quaternäre Verbindung in der Zusammensetzung überführt werden, sie können aber selbstverständlich auch als permanent quaternäre Verbindung in den erfindungsgemäßen Zusammensetzungen verwendet werden. Beispiele für permanent quaternierte Amidoamine sind beispielsweise die Rohstoffe mit der Handelsbezeichnung Rewoquat^{®} UTM 50, Lanoquat^{®} DES-50 oder Empigen CSC.

Die kationischen Tenside (E5) sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Kationische, zwitterionische und/oder amphotere Tenside sowie deren Mischungen können erfindungsgemäß bevorzugt sein. Anionische Tenside werden insbesondere verwendet, wenn die erfindungsgemäßen Zusammensetzungen als Shampoos verwendet werden sollen.

Die Tenside (E) werden in Mengen von 0,05 - 45 Gew.%, bevorzugt 0,1 - 30 Gew.% und ganz besonders bevorzugt von 0,5 - 25 Gew.%, bezogen auf das gesamte erfindungsgemäß verwendete Mittel, eingesetzt.

Erfindungsgemäß verwendbare Emulgatoren sind beispielsweise

Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
Ethylenoxid- und Polyglycerin-Anlagerungsprodukte an Methylglucosid-Fettsäureester, Fettsäurealkanolamide und Fettsäureglucamide,
C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
Gemische aus Alkyl-(oligo)-glucosiden und Fettalkoholen zum Beispiel das im Handel erhältliche Produkt Montanov^{®}68,
Anlagerungsprodukte von 5 bis 60 Mol Ethylenoxid an Rizinusöl und gehärtetes Rizinusöl,
Partialester von Polyolen mit 3-6 Kohlenstoffatomen mit gesättigten Fettsäuren mit 8 bis 22 C-Atomen,
Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.

Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.

Fettsäureester von Zuckern und Zuckeralkoholen, wie Sorbit,
Polyglycerine und Polyglycerinderivate wie beispielsweise Polyglycerinpoly-12-hydroxystearat (Handelsprodukt Dehymuls^{®} PGPH),
Lineare und verzweigte Fettsäuren mit 8 bis 30 C - Atomen und deren Na-, K-, Ammonium-, Ca-, Mg- und Zn - Salze.

Als besonders vorteilhaft hat sich auch ein Zusatz eines an sich bekannten Emulgators vom Typ Wasser-in-Öl in einer Menge von ca. 1 - 5 Gew.-% erwiesen. Dabei handelt es sich um einen Mischester, der ein Kondensationsprodukt aus einem Pentaerythrit-di-fettsäureester und einem Zitronensäure-di-fettalkoholester darstellt, wie es in DE-PS 11 65 574 näher beschrieben ist. Durch den Zusatz solcher Mischester wird ein besonders cremiger, feinblasiger Schaum und ein angenehmes Hautgefühl bei der Anwendung des Körperreinigungsmittels erreicht.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Weiterhin ist es erfindungsgemäß bevorzugt, wenn die milden anionischen Tenside mit anderen als mild oder besonders mild bekannten Tensiden und Emulgatoren gemeinsam verwendet werden. Die Kriterien hierfür sind die bereits zuvor beschriebenen Testmodelle.

Nach diesen Prüfmethoden haben sich die folgenden Tenside und Emulgatoren als mild bis besonders mild erwiesen und sind erfindungsgemäß besonders bevorzugt:
Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
Zwitterionische Tenside (E2),
Ampholytische Tenside (E3),
Zuckertenside vom Typ der Alkyl- oder Alkenyloligoglykoside gemäß der Formel (E4-II),
Zuckertenside vom Typ der Fettsäure-N-alkylpolyhydroxyalkylamide gemäß der Formel (E4-III),
Anlagerungsprodukte von 5 bis 60 Mol Ethylenoxid an Rizinusöl und gehärtetes Rizinusöl,
Polyolfettsäureester, wie beispielsweise das Handelsprodukt Hydagen^{®} HSP (Cognis) oder Sovermol^{®}- Typen (Cognis),
Aminoxide,
Hydroxymischether, wie sie beispielsweise in der DE-OS 19738866 beschrieben sind,
Sorbitanfettsäureester und Anlagerungeprodukte von Ethylenoxid an Sorbitanfettsäureester wie beispielsweise die Polysorbate,
Zuckerfettsäureester und Anlagerungsprodukte von Ethylenoxid an Zuckerfettsäureester,
Anlagerungsprodukte von Ethylenoxid an Fettsäurealkanolamide und Fettamine,
Zuckertenside vom Typ der Alkyl- und Alkenyloligoglykoside gemäß Formel (E4-II),
Esterquats,
Alkylamidoamine und quaternierte Alkylamidoamine.

C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,

Gemische aus Alkyl-(oligo)-glucosiden und Fettalkoholen zum Beispiel das im Handel erhältliche Produkt Montanov^{®}68,

Anlagerungsprodukte von 5 bis 60 Mol Ethylenoxid an Rizinusöl und gehärtetes Rizinusöl,
Partialester von Polyolen mit 3-6 Kohlenstoffatomen mit gesättigten Fettsäuren mit 8 bis 22 C-Atomen,
Sterine, Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.

Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.

Fettsäureester von Zuckern und Zuckeralkoholen, wie Sorbit,
Polyglycerine und Polyglycerinderivate wie beispielsweise Polyglycerinpoly-12-hydroxystearat (Handelsprodukt Dehymuls^{®} PGPH),
In dem erfindungsgemäß bevorzugten Fall, dass die Verträglichkeit der kosmetischen Zusammensetzungen verbessert werden soll, werden die besonders milden Tenside und Emulgatoren in den Zusammensetzungen bevorzugt verwendet. In diesen Fällen werden die Alkylsulfate und / oder Alkylethersulfate in Mengen unterhalb von 8 Gew.% , bevorzugt kleiner 5 Gew.% und besonders bevorzugt kleiner 2,5 Gew.% verwendet. Ganz besonders bevorzugt sind diese Zusammensetzungen frei von Alkylsulfaten und / oder Alkylethersulfaten. "Frei von" bedeutet in diesem Zusammenhang, dass diese Inhaltsstoffe keinesfalls zusätzlich verwendet werden. Es ist jedoch möglich, dass sie durch andere Inhaltsstoffe, wie beispielsweise durch die Verwendung von Silikonemulsionen, in die Zusammensetzung gelangen. Vorzugsweise bedeutet "frei von" daher auch kleiner als 0,5 Gew.%, besonders bevorzugt kleiner 0,1 Gew.%. Weiterhin bedeutet "frei von" in diesem Zusammenhang nicht, dass die erfindungsgemäßen Zusammensetzungen keine in der Homologenverteilung eingeschränkten Fettalkoholethersulfate enthalten dürfen. Dies ist vielmehr eine Ausnahme, da diese anionischen Tenside den milden anionischen Tensiden zuzurechnen sind. Erfindungsgemäß am bevorzugtesten ist jedoch die ausschließliche Verwendung von milden Tensiden. Sollten in diesen Fällen als Beimengung zu anderen Inhaltsstoffen "nicht milde Tenside" diese in die erfindungsgemäßen Zusammensetzungen gelangen, so gelten die zuvor genannten Mengen für diese Beimengungen.

Zu den Synergisten der erfindungsgemäßen Zusammensetzungen zählen anionische sowie nichtionische Polymere.

Im Folgenden werden einige Beispiele von besonders bevorzugten Polymeren beschrieben.

Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfon-säure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Die anionischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Eine weitere ganz besonders bevorzugte Gruppe von Polymeren sind Polyurethane. Die Polyurethane bestehen aus mindestens zwei verschiedenen Monomertypen,
- einer Verbindung (V1) mit mindestens 2 aktiven Wasserstoffatomen pro Molekül und
- einem Di- oder Polyisocyanat (V2).

Beispiele für Verbindungen (V1) sind Ethylenglykol, 1,2- und 1,3-Propylenglykol, Butylenglykole, Di-, Tri-, Tetra- und Poly-Ethylen- und -Propylenglykole, Copolymere von niederen Alkylenoxiden wie Ethylenoxid, Propylenoxid und Butylenoxid, Ethylendiamin, Propylendiamin, 1,4-Diaminobutan, Hexamethylendiamin und α,ω-Diamine auf Basis von langkettigen Alkanen oder Polyalkylenoxiden.

Polyurethane, bei denen die Verbindungen (V1) Diole, Triole und Polyetherole sind, können erfindungsgemäß bevorzugt sein. Insbesondere Polyethylenglykole und Polypropylenglykole mit Molmassen zwischen 200 und 3000, insbesondere zwischen 1600 und 2500, haben sich in einzelnen Fällen als besonders geeignet erwiesen.

Als Verbindungen (V2) werden überwiegend Hexamethylendiisocyanat, 2,4- und 2,6-Toluoldiisocyanat, 4,4'-Methylendi(phenylisocyanat) und insbesondere Isophorondiisocyanat eingesetzt.

Ein erfindungsgemäß ganz besonders bevorzugtes Polyurethan ist unter der Handelsbezeichnung Luviset^{®} PUR (BASF) im Handel.

Die erfindungsgemäßen Mittel können in einer weiteren Ausführungsform nichtionogene Polymere (G4) enthalten.

Geeignete nichtionogene Polymere sind beispielsweise:
Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinyl-acetat-Copolymere, sind ebenfalls bevorzugte nichtionische Polymere.

Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.

Stärke und deren Derivate, insbesondere Stärkeether, beispielsweise Structure^{®} XL (National Starch), eine multifunktionelle, salztolerante Stärke;

### - Schellack

Polyvinylpyrrolidone, wie sie beispielsweise unter der Bezeichnung Luviskol^{®} (BASF) vertrieben werden.

Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.

Glycosidisch substituierte Silicone.

Die nichtionischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Es ist erfindungsgemäß auch möglich, daß die verwendeten Zubereitungen mehrere, insbesondere zwei verschiedene Polymere gleicher Ladung und/oder jeweils ein ionisches und ein amphoteres und/oder nicht ionisches Polymer enthalten.

Weitere bevorzugte Polymere sind alle Polymere, welche im "International Cosmetic Ingredient Dictionary and Handbook", (seventh edition 1997, The Cosmetic, Toiletry, and Fragrance Association 1101 17th Street, N.W., Suite 300, Washington, DC 20036-4702) als Polymere in einem der Kapitel über Polymere wie beispielsweise "film formers" oder "hair fixatives" genannt und im Handel erhältlich sind. Auf diese Schrift und die daraus zitierten Abschnitte wird ausdrücklich Bezug genommen.

Mit besonderem Vorzug enthalten die erfindungsgemäßen Zusammensetzungen Fettstoffe (D) als weiteren Wirkstoff. In einer besonders bevorzugen Ausführungsform enthält der erfindungsgemäße Wirkstoffkomplex einen Sandelholzextrakt, einen Fettstoff und einen weiteren Wirkstoff ausgewählt aus den kationischen Tensiden, den amphoteren und/oder zwitterionischen Tensiden, den kationischen Polymeren oder den amphoteren und/oder zwitterionischen Polymeren. Durch diese Wirkstoffkombination werden erhöhte Mengen der Wirkstoffe auf dem Haar oder der Haut abgeschieden, was zu synergistisch gesteigerten Effekten führt.

Unter Fettstoffen (D) sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen.

Als Fettsäuren (D1) können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. Bevorzugt beträgt die Menge 0,5 - 10 Gew.%, wobei ganz besonders vorteilhaft Mengen von 1 - 5 Gew.% sein können.

Als Fettalkohole (D2) können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab. Erfindungsgemäß einsetzbar sind ebenfalls solche Fettalkoholschnitte, die ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 - 20 Gew.-% eingesetzt.

Als natürliche oder synthetische Wachse (D3) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Zu den natürlichen und synthetischen kosmetischen Ölkörpern (D4) sind beispielsweise zu zählen:
pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.

Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Erfindungsgemäß besonders bevorzugt sind Isopropylmyristat (Riianit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).

Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
Trifettsäureester von gesättigten und/oder ungesättigten linearen und/oder verzweigten Fettsäuren mit Glycerin,
Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (D4-I), 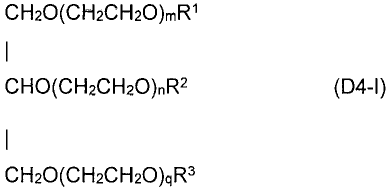 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, daß mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäß verwendeten Mitteln beträgt üblicherweise 0,1 - 30 Gew.%, bezogen auf das gesamte Mittel, bevorzugt 0,1 - 20 Gew.-%, und insbesondere 0,1 - 15 Gew.-%.

Eine letzte Substanzgruppe, welche als Fettstoffe verwendet werden können, sind Silikone.

Als weitere Stoffklasse, welche alternativ zu den zuvor beschriebenen in den erfindungsgemäßen Mitteln als Wirkstoff enthalten ist, sind die Silikonöle (S). Zunächst werden hierunter die Dimethiconole (S1) verstanden. Dimethiconole bilden die erste Gruppe der Silikone, welche erfindungsgemäß besonders bevorzugt sind. Die erfindungsgemäßen Dimethiconole können sowohl linear als auch verzweigt als auch cyclisch oder cyclisch und verzweigt sein. Lineare Dimethiconole können durch die folgende Strukturformel (S1 - I) dargestellt werden:

(SiOHR¹₂) - O - (SiR²₂ - O - )ₓ - (SiOHR¹₂) (S1 - I)

Verzweigte Dimethiconole können durch die Strukturformel (S1 - II) dargestellt werden:

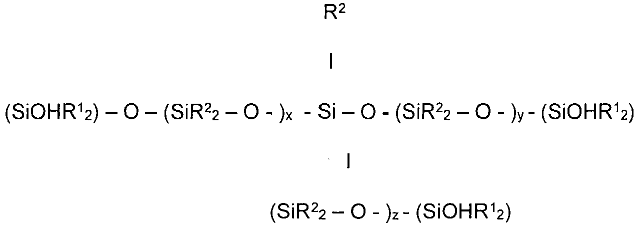

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, -CH₂CH₂OCH₂-, -OCH₂CH₂-, -OCH₂ CH₂CH₂-, -CH₂CH(CH₃)C(O)OCH₂-,-(CH₂)₃ CC(O)OCH₂CH₂-, -C₆H₄C₆H₄-, -C₆H₄CH₂C₆H₄-; und -(CH₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, banz besonders bevorzugte Vsikositäten liegen zwischen 10000 und 3000000 cPs. Der bevorzugteste Bereich liegt zwischen 50000 und 2000000 cPs.

Selbstverständlich umfasst die erfindungsgemäße Lehre auch, dass die Dimethiconole bereits als Emulsion vorliegen können. Dabei kann die entsprechende Emulsion der Dimethiconole sowohl nach der Herstellung der entsprechenden Dimethiconole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole auch direkt durch ein Emulsionspolymerisationsverfahren hergestellt werden. Auch derartige Verfahren sind dem Fachmann wohl bekannt. Hierzu sei beispielsweise verwiesen auf die "Encyclopedia of Polymer Science and Engineering, Volume 15, Second Edition, Seiten 204 bis 308, John Wiley & Sons, Inc. 1989. Auf dieses Standardwerk wird ausdrücklich Bezug genommen.

Wenn die erfindungsgemäßen Dimethiconole als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt.

Werden verzweigte Dimethiconole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole ganz besonders bevorzugt sein.

Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS ( beide Guardian Laboratories), Nonychosine E, Nonychosine V (beide Exsymol), SanSurf Petrolatum-25, Satin Finish ( beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Die Dimethiconole (S1) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconol bezogen auf die Zusammensetzung.

Erfindungsgemäß ist es auch möglich, dass die Dimethiconole eine eigene Phase in den erfindungsgemäßen Zusammensetzungen bilden. In diesem Fall kann es angebracht sein, wenn die Zusammensetzung unmittelbar vor der Anwendung durch Schütteln kurzfristig homogenisiert wird. In diesem Falle kann die Menge an Dimethiconol bis zu 40 Gew.%, bevorzugt in Mengen von bis zu 25 Gew.%bezogen auf die Gesamtzusammensetzung betragen.

Dimethicone (S2) bilden die zweite Gruppe der Silikone, welche erfindungsgemäß besonders bevorzugt sind. Die erfindungsgemäßen Dimethicone können sowohl linear als auch verzweigt als auch cyclisch oder cyclisch und verzweigt sein. Lineare Dimethicone können durch die folgende Strukturformel (S2 - I) dargestellt werden:

(SiR¹₃) - O - (SiR²₂ - O - )ₓ - (SiR¹₃) (S2 - I)

Verzweigte Dimethicone können durch die Strukturformel (S2 - II) dargestellt werden:

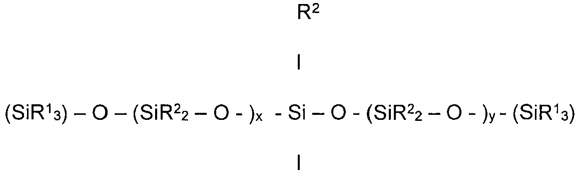

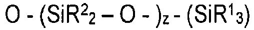

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, -CH₂CH₂OCH₂-, -OCH₂CH₂-, -OCH₂ CH₂CH₂-, -CH₂CH(CH₃)C(O)OCH₂-, - (CH₂)₃CC(O)OCH₂CH₂-, -C₆H₄C₆H₄-, -C₆H₄CH₂C₆H₄-; und -(CH₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, banz besonders bevorzugte Vsikositäten liegen zwischen 10000 und 3000000 cPs. Der bevorzugteste Bereich liegt zwischen 50000 und 2000000 cPs.

Selbstverständlich umfasst die erfindungsgemäße Lehre auch, dass die Dimethicone bereits als Emulsion vorliegen können. Dabei kann die entsprechende Emulsion der Dimethicone sowohl nach der Herstellung der entsprechenden Dimethicone aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethicone auch direkt durch ein Emulsionspolymerisationsverfahren hergestellt werden. Auch derartige Verfahren sind dem Fachmann wohl bekannt. Hierzu sei beispielsweise verwiesen auf die "Encyclopedia of Polymer Science and Engineering, Volume 15, Second Edition, Seiten 204 bis 308, John Wiley & Sons, Inc. 1989. Auf dieses Standardwerk wird ausdrücklich Bezug genommen.

Wenn die erfindungsgemäßen Dimethicone als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt.

Werden verzweigte Dimethicone verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethicone ganz besonders bevorzugt sein.

Die Dimethicone (S2) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconon bezogen auf die Zusammensetzung.

Erfindungsgemäß ist es auch möglich, dass die Dimethicone eine eigene Phase in den erfindungsgemäßen Zusammensetzungen bilden. In diesem Fall kann es angebracht sein, wenn die Zusammensetzung unmittelbar vor der Anwendung durch Schütteln kurzfristig homogenisiert wird. In diesem Falle kann die Menge an Dimethicon bis zu 40 Gew.%, bevorzugt in Mengen von bis zu 25 Gew.% bezogen auf die Gesamtzusammensetzung betragen.

Dimethiconcopolyole (S3) bilden eine weitere Gruppe bevorzugter Silikone. Dimethiconcopolyole können durch die folgende Strukturformeln dargestellt werden:

(SiR¹₃)-O-(SiR²₂-O-)ₓ- (SiRPE-O-)_{y}-(SiR¹₃) (S3 - I)

oder durch die nachfolgende Strukturformel:

PE - (SiR¹₂)-O-(SiR²₂-O-)ₓ-(SiR¹₂) - PE (S3 - II)

Verzweigte Dimethiconcopolyole können durch die Strukturformel (S3-III) dargestellt werden: 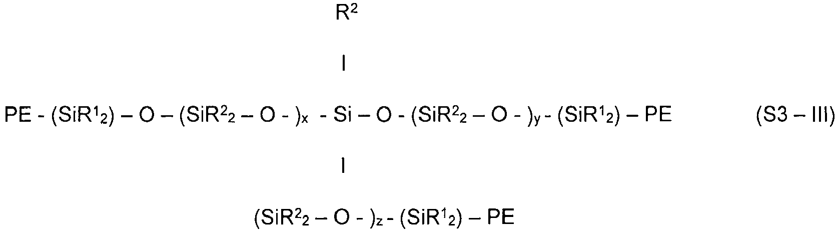 oder durch die Strukturformel(S3 - IV):

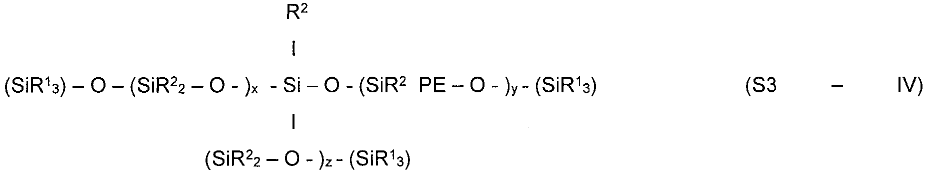

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, -CH₂CH₂OCH₂-, -OCH₂CH₂-, -OCH₂ CH₂CH₂-, -CH₂CH(CH₃)C(O)OCH₂-, - (CH₂)₃CC(O)OCH₂CH₂-, -C₆H₄C₆H₄-, -C₆H₄CH₂C₆H₄-; und -(CH₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. PE steht für einen Polyoxyalkylenrest. Bevorzugte Polyoxyalkylenreste leiten sich ab von Ethylenoxid, Propylenoxid und Glycerin. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Selbstverständlich umfasst die erfindungsgemäße Lehre auch, dass die Dimethiconcopolymere bereits als Emulsion vorliegen können. Dabei kann die entsprechende Emulsion der Dimethiconcopolyole sowohl nach der Herstellung der entsprechenden Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconcopolyole auch direkt durch ein Emulsionspolymerisationsverfahren hergestellt werden. Auch derartige Verfahren sind dem Fachmann wohl bekannt. Hierzu sei beispielsweise verwiesen auf die "Encyclopedia of Polymer Science and Engineering, Volume 15, Second Edition, Seiten 204 bis 308, John Wiley & Sons, Inc. 1989. Auf dieses Standardwerk wird ausdrücklich Bezug genommen.

Wenn die erfindungsgemäßen Dimethiconcopolyole als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt.

Werden verzweigte Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconcopolyole ganz besonders bevorzugt sein.

Die Dimethiconcopolyole (S3) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconcopolyol bezogen auf die Zusammensetzung.

Erfindungsgemäß ist es auch möglich, dass die Dimethiconcopolyole eine eigene Phase in den erfindungsgemäßen Zusammensetzungen bilden. In diesem Falle kann die Menge an Dimethiconcopolyol bis zu 40 Gew.%, bevorzugt in Mengen von bis zu 25 Gew.% bezogen auf die Gesamtzusammensetzung betragen.

Aminofunktionelle Silikone oder auch Amodimethicone (S4) genannt, sind Silicone, welche mindestens eine (gegebenenfalls substituierte) Aminogruppe aufweisen.

Solche Silicone können z.B. durch die Formel (S4 - I)

M(RₐQ_{b}SiO_{(4-a-b)/2)x}(R_{c}SiO_{(4-c)/2)y}M (S4 - I)

beschreiben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-,-CH₂CH₂OCH₂-, -OCH₂CH₂-, -OCH₂ CH₂CH₂-, -CH₂CH(CH₃)C(O)OCH₂-, -(CH₂)₃CC(O)OCH₂CH₂-, -C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den RcSiO _{(4-c)/2}-Einheiten liegt im Bereich von etwa 1 : 2 bis 1 : 65, vorzugsweise von etwa 1:5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße Mittel sind dadurch gekennzeichnet, daß sie ein aminofunktionelles Silikon der Formel (S4 - II)

R'ₐG₃₋ₐ-Si(OSiG₂)ₙ-(OSiG_{b}R'_{2-b})ₘ-O-SiG₃₋ₐ-R'ₐ (S4 - II),

enthalten, worin bedeutet:
G ist-H, eine Phenylgruppe, -OH, -O-CH₃, -CH₃, -CH₂CH₃, -CH₂CH₂CH₃, -CH(CH₃)₂,-CH₂CH₂CH₂H₃, -CH₂CH(CH₃)₂, -CH(CH₃)CH₂CH₃, -C(CH₃)₃;
a steht für eine Zahl zwischen 0 und 3, insbesondere 0;
b steht für eine Zahl zwischen 0 und 1, insbesondere 1,
m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
R' ist ein monovalenter Rest ausgewählt aus
-N(R")-CH₂-CH₂-N(R")₂
-N(R")₂
-N⁺(R")₃A⁻
-N⁺H(R")₂ A⁻
-N⁺H₂(R")A⁻
-N(R")-CH₂-CH₂-N⁺R"H₂A⁻,
wobei jedes R" für gleiche oder verschiedene Reste aus der Gruppe -H, -Phenyl, -Benzyl, der C₁₋₂₀-Alkylreste, vorzugsweise -CH₃, -CH₂CH₃, -CH₂CH₂CH₃, -CH(CH₃)₂, -CH₂CH₂CH₂H₃,-CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Besonders bevorzugte erfindungsgemäße Mittel sind dadurch gekennzeichnet, daß sie ein aminofunktionelles Silikon der Formel (S4 - III) 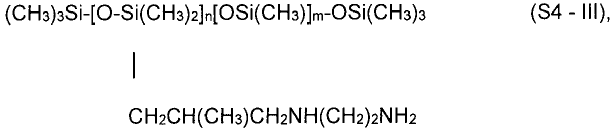 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Besonders bevorzugt sind auch erfindungsgemäße Mittel, die dadurch gekennzeichnet sind, daß sie ein aminofunktionelles Silikon der Formel (S4 - IV) 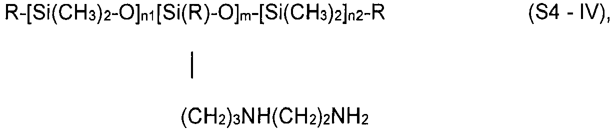 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCl-Deklaration als Amodimethicone bezeichnet.

Unabhängig davon, welche aminofunktionellen Silicone eingesetzt werden, sind erfindungsgemäße Mittel bevorzugt, bei denen das aminofunktionelle Silikon eine Aminzahl oberhalb von 0,25 meq/g, vorzugsweise oberhalb von 0,3 meq/g und insbesondere oberhalb von 0,4 meq/g aufweist. Die Aminzahl steht dabei für die Milli-Äquivalente Amin pro Gramm des aminofunktioinelen Silicons. Sie kann durch Titration ermittelt und auch in der Einheit mg KOH/g angegeben werden.

Die Amodimethicone (S4) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Amodimethicon bezogen auf die Zusammensetzung.

Erfindungsgemäß ist es auch möglich, dass die Amodimethicone eine eigene Phase in den erfindungsgemäßen Zusammensetzungen bilden. In diesem Fall kann es angebracht sein, wenn die Zusammensetzung unmittelbar vor der Anwendung durch Schütteln kurzfristig homogenisiert wird. In diesem Falle kann die Menge an Amodimethicon bis zu 40 Gew.%, bevorzugt in Mengen von bis zu 25 Gew.% bezogen auf die Gesamtzusammensetzung betragen.

Erst seit kurzem sind völlig neuartige Polyammmonium-Polysiloxan Verbindungen bekannt, in welchen die Siloxansubstrukturen gegebenenfalls über Ammoniumsubstrukturen miteinander verbunden sind. Derartige Verbindungen und deren Verwendung in kosmetischen Mitteln werden beispielsweise in der Offenlegungsschrift WO 02/10257 beschrieben.

Als Silikon können die erfindungsgemäßen Zusammensetzungen mindestens eine Polyammonium-Polysiloxan Verbindung enthalten:
Die vorstehend beschriebenen Polyammonium-Polysiloxan Verbindungen können beispielsweise unter der Handelsbezeichnung Baysilone^{®} von GE Bayer Silicones bezogen werden. Die Produkte mit den Bezeichnungen Baysilone TP 3911, SME 253 und SFE 839 sind dabei bevorzugt. Ganz besonders bevorzugt ist die Verwendung von Baysilone TP 3911 als Wirkkomponente der erfindungsgemäßen Zusammensetzungen.
Die vorstehend beschriebenen Polyammonium-Polysiloxan Verbindungen werden in dem erfindungsgemäßen Zusammensetzungen in einer Menge von 0,01 bis 10 Gew.% , vorzugsweise 0,01 bis 7,5, besonders bevorzugt 0,01 bis 5,0 Gew.%, ganz besonders bevorzugt von 0,05 bis 2,5 Gew.% jeweils in Bezug auf die Gesamtzusammensetzung verwendet.

Das Verhältnis der Polyammonium-Polysiloxan Verbindungen zu einer weiteren synergistischen Wirkstoffkomponente ausgewählt aus der Gruppe der Polymere, der Proteinhydrolysate, der Silikone, der Vitamine und der Pflanzenextrakte beträgt im allgemeinen erfindungsgemäß 1:1000 bis 1:2, vorzugsweise 1:100 bis 1:2, besonders bevorzugt 1:50 bis 1:2 und ganz besonders bevorzugt 1:10 bis 1:2.

Wenn eine Mischung aus mindestens zwei Silikonen verwendet wird, so ist diese Mischung in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Silikonmischung bezogen auf die Zusammensetzung enthalten.

Erfindungsgemäß ist es auch möglich, dass die Mischung der Silikone eine eigene Phase in den erfindungsgemäßen Zusammensetzungen bilden. In diesem Fall kann es angebracht sein, wenn die Zusammensetzung unmittelbar vor der Anwendung durch Schütteln kurzfristig homogenisiert wird. In diesem Falle kann die Menge an Silikonmischung bis zu 40 Gew.%, bevorzugt in Mengen von bis zu 25 Gew.%bezogen auf die Gesamtzusammensetzung betragen.

Selbstverständlich umfasst die erfindungsgemäße Lehre auch, dass eine Mischung aus mehreren Fettstoffen (D) aus unterschiedlichen Klassen von Fettstoffen, mindestens zwei unterschiedlichen Fettstoffklassen in den erfindungsgemäßen Zusammensetzungen verwendet werden kann. Die bevorzugten Mischungen aus mindestens zwei Öl- und Fettkomponenten enthalten in diesem Falle zwingend mindestens eine weitere Silikonkomponente. Bevorzugt wird die Silikonkomponente in diesem Falle ausgewählt aus den Dimethiconolen und den Amodimethiconen.

Die Gesamtmenge an Öl- und Fettkomponenten in den erfindungsgemäßen Mitteln beträgt üblicherweise 0,5 - 75 Gew.-%, bezogen auf das gesamte Mittel. Mengen von 0,5 - 35 Gew.-% sind erfindungsgemäß bevorzugt.

Ein weiterer erfindungsgemäßer synergistischer Wirkstoff in dem erfindungsgemäßen Wirkstoffkomplex mit einem Sandelholzextrakt sind Proteinhydrolysate und/oder dessen Derivate (P).

Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton. Selbstverständlich umfasst die vorliegende erfindungsgemäße Lehre auch, dass im Falle der Aminosäuren diese in Form von Derivaten, wie beispielsweise der N-Acylderivate, der N-Alkyl oder der O-Ester vorliegen können. Im Falle der N-acylderivate ist die Acylgruppe eine Formylrest, ein Acetylrest, ein Propionylrest, ein Butyrylrest oder der Rest einer geradkettigen, verzweigten oder unverzweigten, gesättigten oder ungesättigten Fettsäure mit einer Kettenlänge von 8 bis 30 C-Atomen. Im Falle einer N-Alkylderivate kann die Alkylgruppe linear, verzweigt, gesättigt oder ungesättigt sein und hat eine C-Kettenlänge von 1 bis 30 C-Atomen. Im Falle der O-Ester sind die der Veresterung zugrunde liegenden Alkohole Methanol, Ethanol, Isopropanol, Propanol, Butanol, Isobutanol, Pentanol, Neopentanol, Isopentanol, Hexanole, Heptanole, Capryl- oder Capronalkohol, Octanole, Nonanole, Decanole, Dodecanole, Lauranole, insbesondere gesättigte oder ungesättigte, lineare oder verzweigte Alkohole mit einer C-Kettenlänge von 1 bis 30 C-Atomen. Selbstverständlich können die Aminosäuren sowohl am N-Atom als auch am O-Atom gleichzeitig derivatisiert sein. Selbstverständlich können die Aminosäuren auch in Salzform, insbesondere als Mischsalze zusammen mit Genusssäuren verwendet werden. Dies kann erfindungsgemäß bevorzugt sein.

Als Beispiele für Aminosäuren und deren Derivaten als erfindungsgemäße Proteinhydrolysate werden genannt: Alanin, Arginin, Carnitin, Creatin, Cystathionin, Cystein, Cystin, Cystinsäure, Glycin, Histidin, Homocystein, Homoserin, Isoleucin, Lanthionin, Leucin, Lysin, Methionin, Norleucin, Norvalin, Ornithin, Phenylalanin, Prolin, Hydroxyprolin, Sarcosin, Serin, Threonin, Tryptophan, Thyronin, Tyrosin, Valin, Asparaginsäure, Asparagin, Glutaminsäure und Glutamin. Bevorzugte Aminosäuren sind Alanin, Arginin, Glycin, Histidin, Lanthionin, Leucin, Lysin, Prolin, Hydroxyprolin Serin und Asparagin. Ganz besonders bevorzugt werden verwendet Alanin, Glycin, Histidin, Lysin, Serin und Arginin. Am bevorzugtesten werden Glycin, Histidin, Lysin und Serin verwendet.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

In jüngster Zeit werden immer häufiger insbesondere Pflanzenproteine und deren Hydrolysate sowie Derivate in der Kosmetik verwendet. Bekannt sind beispielsweise Produkte auf der Basis von Weizen, Hafer, Reis, Mais, Kartoffeln oder Soja. Zu den Pflanzen, welche interessante wirksame Inhaltsstoffe enthalten, gehört auch die Familie der Moringagewächse. Ein derartiges Protein ist beispielsweise unter der Handelsbezeichnung Puricare^{®} LS 9658 von der Fa. Laboratoires Sérobiologiques im Handel erhältlich.

Weiterhin sind erfindungsgemäß bevorzugte pflanzliche Proteinhydrolysaten wie beispielsweise Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Weitere erfindungsgemäß bevorzugte Proteinhydrolysate sind maritimen Ursprunges. Hierzu zählen beispielsweise Kollagenhydrolysate von Fischen oder Algen sowie Proteinhydrolysate von Muscheln bzw. Perlenhydrolysate.

Beispiele für erfindungsgemäße Perlenextrakte sind die Handelsprodukte Pearl Protein Extract BG^{®} oder Crodarom^{®} Pearl.

In den kosmetischen Zusammensetzungen ist einer der zuvor beschriebenen Perlenextrakte in einer Menge von mindestens 0,01 bis zu 20 Gew.% enthalten. Bevorzugt werden Mengen des Extraktes von 0,01 bis zu 10 Gew.%, ganz besonders bevorzugt Mengen von 0,01 bis 5 Gew.% bezogen auf die gesamte kosmetische Zusammensetzung verwendet.

Ein weiteres ganz besonderes Proteinhydrolysat wird aus der Seide gewonnen.

So wird beispielsweise Sericin als solches seitens der Fa. Pentapharm Ltd. als Handelsprodukt mit der Bezeichnung Sericin Code 303-02 vertrieben. Weitaus häufiger noch wird Fibroin als Proteinhydrolysat mit unterschiedlichen Molekulargewichten im Markt angeboten. Diese Hydrolysate werden insbesondere als "Seidenhydroylsate" verstanden. So wird beispielsweise unter der Handelsbezeichnung Promois^{®} Silk hydrolysiertes Fibroin mit mittleren Molekulargewichten zwischen 350 und 1000 vertrieben.

Erfindungsgemäß bevorzugt können als Wirkstoffe verwendet werden:
natives Sericin,
hydrolysiertes und/oder weiter derivatisiertes Sericin, wie beispielsweise Handelsprodukte mit den INCl - Bezeichnungen Sericin, Hydrolyzed Sericin, oder Hydrolyzed Silk,
eine Mischung aus den Aminosäuren Serin, Aspartat und Glycin und/oder deren Methyl, Propyl, iso-Propyl, Butyl, iso-Butylestern, deren Salze wie beispielsweise Hydrochloride, Sulfate, Acetate, Citrate, Tartrate , wobei in dieser Mischung das Serin und/oder dessen Derivate zu 20 bis 60 Gew.%, das Aspartat und/oder dessen Derivate zu 10 - 40 Gew.% und das Glycin und/oder dessen Derivate zu 5 bis 30 Gew.% enthalten sind, mit der Maßgabe, daß sich die Mengen dieser Aminosäuren und/oder deren Derivate vorzugsweise zu 100 Gew.% ergänzen,
sowie deren Mischungen.

Erfindungsgemäß können als Wirkstoffe weiterhin verwendet werden:
natives, in eine lösliche Form überführtes Fibroin,
hydrolysiertes und/oder weiter derivatisiertes Fibroin, besonders teilhydrolisiertes Fibroin, welches als Hauptbestandteil die Aminosäuresequenz Ser-Gly-Ala-Gly-Ala-Gly enthält,
die Aminosäuresequenz Ser-Gly-Ala-Gly-Ala-Gly,
eine Mischung der Aminosäuren Glycin, Alanin und Tyrosin und/oder deren Methyl, Propyl, iso-Propyl, Butyl, iso-Butylestern, deren Salze wie beispielsweise Hydrochloride, Sulfate, Acetate, Citrate, Tartrate , wobei in dieser Mischung das Glycin und/oder dessen Derivate in Mengen von 20 - 60 Gew.%, das Alanin und dessen Derivate in Mengen von 10 - 40 Gew,% und das Tyrosin und dessen Derivate in Mengen von 0 bis 25 Gew.% enthalten sind, mit der Maßgabe, daß sich die Mengen dieser Aminosäuren und/oder deren Derivate vorzugsweise zu 100 Gew.% ergänzen,
sowie deren Mischungen.

Wenn in den erfindungsgemäßen Zusammensetzungen des erfindungsgemäßen Mittels gleichzeitig beide Seidenproteinhydrolysate und/oder deren Derivate verwendet werden, kann es erfindungsgemäß bevorzugt sein, daß mindestens eine der beiden Seidenbestandteile, Fibroin oder Sericin in der nativen oder allenfalls löslich gemachten Form verwendet wird. Erfindungsgemäß ist es auch möglich, eine Mischung aus mehreren Seidenproteinhydrolysaten und/oder deren Derivaten einzusetzen.

Wenn eine Mischung aus mindestens zwei Seidenhydrolysaten und/oder deren Derivaten verwendet wird, kann es erfindungsgemäß bevorzugt sein, daß die beiden Seidenproteinhydrolysate im Verhältnis von 10:90 bis 70:30, insbesondere 15:85 bis 50:50 und ganz besonders 20:80 bis 40:60 bezogen auf deren jeweilige Gehalte an aktiver Wirksubstanz in den erfindungsgemäßen Zubereitungen eingesetzt werden.

Die Derivate der Hydrolysate von Sericin und Fibroin umfassen sowohl anionische als auch kationisierte Proteinhydrolysate. Als typische Beispiele für die erfindungsgemäßen kationischen Proteinhydrolysate und -derivate seien die unter den INCl - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Silk, Cocodimonium Hydroxypropyl Silk Amino Acids, Hydroxyproypltrimonium Hydrolyzed Silk, Lauryldimonium Hydroxypropyl Hydrolyzed Silk, Steardimonium Hydroxypropyl Hydrolyzed Silk, Quaternium-79 Hydrolyzed Silk. Als typische Beispiele für die erfindungsgemäßen anionischen Proteinhydrolysate und -derivate seien die unter den INCl - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Potassium Cocoyl Hydrolyzed Silk, Sodium Lauroyl Hydrolyzed Silk oder Sodium Stearoyl Hydrolyzed Silk. Letztlich seien noch als typische Beispiele für die erfindungsgemäß einsetzbaren Derivate aus Sericin und Fibroin die unter den INCl - Bezeichnungen im Handel erhältlichen Produkte genannt: Ethyl Ester of Hydrolyzed Silk und Hydrolyzed Silk PG-Propyl Methylsilanediol. Weiterhin erfindungsgemäß verwendbar, wenngleich nicht unbedingt bevorzugt sind die im Handel erhältlichen Produkte mit den INCl - Bezeichnungen Palmitoyl Oligopeptide, Palmitoyl Pentapeptide-3, Palmitoyl Pentapeptide-2, Acetyl Hexapeptide-1, Acetyl Hexapeptide-3, CopperTripeptide-1, Hexapeptide-1, Hexapeptide-2, MEA-Hydrolyzed Silk.

In den erfindungsgemäß verwendeten Mitteln sind die Seidenproteinhydroysate und/oder deren Derivate in Mengen von 0,001 - 10 Gew.-% bezogen auf das gesamte Mittel enthalten. Mengen von 0,005 bis 5, insbesondere 0,01 bis 3 Gew.-%, sind ganz besonders bevorzugt.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda) oder Crotein^{®} (Croda) vertrieben.

Eine weitere Gruppe von erfindungsgemäßen Proteinhydrolysaten sind daher kationisch derivatisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Als typische Beispiele für die erfindungsgemäßen kationischen Proteinhydrolysate und - derivate seien die unter den INCl - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Selbstverständlich umfaßt die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten (P) einzusetzen.

Die Proteinhydrolysate (P) sind in den Mitteln in Konzentrationen von 0,001 Gew.% bis zu 20 Gew.%, vorzugsweise von 0,05 Gew.% bis zu 15 Gew.% und ganz besonders bevorzugt in Mengen von 0,05 Gew.% bis zu 5 Gew.% enthalten.

Ein weiterer Gegenstand der Erfindung ist daher die Verwendung von Derivaten der 2-Pyrrolidinon-5-carbonsäure. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf das gesamte Mittel, besonders bevorzugt 0,1 bis 5, und insbesondere 0,1 bis 3 Gew.%.

Eine weitere bevorzugte Gruppe von Inhaltsstoffen, welche in hervorragender Weise mit der erfindungsgemäßen Zusammensetzung verwendet werden kann, sind Vitamine, Provitamine oder Vitaminvorstufen.

Vitamine, Pro-Vitamine und Vitaminvorstufen sind dabei besonders bevorzugt, die den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
Vitamin B₁ (Thiamin)
Vitamin B₂ (Riboflavin)
Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
Vitamin B₆ (Pyridoxin sowie Pyridoxamin und Pyridoxal).
Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.
Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.
Vitamin F. Unter dem Begriff "Vitamin F" werden üblicherweise essentielle Fettsäuren, insbesondere Linolsäure, Linolensäure und Arachidonsäure, verstanden.
Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Mittel Vitamine, Provitamine und Vitaminvorstufen aus den Gruppen A, B, E und H. Panthenol, Pantolacton, Pyridoxin und seine Derivate sowie Nicotinsäureamid und Biotin sind besonders bevorzugt.

Die erfindungsgemäßen Zusammensetzungen zusätzlich antimikrobielle Verbindungen enthalten. Geeignete antimikrobielle Verbindungen sind z. B. kationische oberflächenaktive Stoffe wie z. B. Cetyltrimethylammoniumbromid, Benzethoniumchlorid, Cetylpyridiniumchlorid oder das als Aminfluorid bekannte N,N,N'-tris-(2-Hydroxyethyl)-N'-octadecyl-1,3-diaminopropan-dihydrofluorid. Gut eignen sich auch die antimikrobiell wirksamen Biguanidverbindungen wie z. B. das Polyhexamethylenbiguanid (Vantocil® IB, ICI) oder das 1,1'-Hexamethylen-bis-(4-Chlorphenyl)-biguanid ("Chlorhexidin") in Form eines wasserlöslichen, verträglichen Salzes, z. B. in Form des Acetats oder Gluconats. Bevorzugt eignen sich auch die antimikrobiellen 5-Amino-hexahydropyrimidine, z. B. das 1,3-Bis-(2-ethylhexyl)-5-methyl-5-amino-hexahydropyrimidin ("Hexetidin"). Weitere bevorzugt geeignete antimikrobielle Wirkstoffe sind die nichtkationischen, phenolischen, antimikrobiellen Stoffe, insbesondere die halogenierten Phenole und Diphenylether. Besonders geeignete antimikrobielle Verbindungen dieses Types sind z. B. das 6,6'- Methylen-Bis-(2-brom-4-chlorphenol) ("Bromchlorophen") und der 2,4,4'-Trichtor-2'-hydroxydiphenylether ("Triclosan").

Weitere geeignete antimikrobielle Stoffe sind die p-Hydroxybenzoesäureester und Sesquiterpenalkohole wie z. B. das Bisabolol, das Farnesol, das Santalol oder das Nerolidol.

Schließlich ergeben sich durch die Verwendung von Pflanzenextrakten (L) in den erfindungsgemäßen Zusammensetzungen weitere synergistische Vorteile.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Baldrian, Wiesenschaumkraut, Quendel, Schafgarbe, Thymian, Melisse, Hauhechel, Huflattich, Eibisch, Meristem, Ginseng, Kaffee, Kakao, Moringa und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Baldrian, Kaffee, Kakao, Moringa, Hauhechel, Meristem, Ginseng und Ingwerwurzel.

Ganz besonders für die erfindungsgemäßen Zusammensetzung geeignet sind die Extrakte aus Grünem Tee, Mandel, Aloe Vera, Kokosnuß, Mango, Aprikose, Limone, Weizen, Kiwi und Melone.

Weiterhin kann es bevorzugt sein, in den erfindungsgemäßen Mitteln Mischungen aus mehreren, insbesondere aus zwei, verschiedenen Pflanzenextrakten einzusetzen.

Neben den Pflanzenextrakten wird in jüngster Zeit in kosmetischen Zusammensetzungen auch ein Bergkristallextrakt verwendet. Ein Beispiel für einen käuflich verfügbaren Quarzextrakt ist unter der Bezeichnung Crodarom^{®} Rock Crystal von der Firma Croda frei im Handel verfügbar.

Zusätzlich kann es sich als vorteilhaft erweisen, wenn in den erfindungsgemäßen Zusammensetzungen Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Schließlich zeigen experimentelle Befunde, dass die erfindungsgemäßen Zusammensetzungen besonders gut geeignet sind, um Parfümöle oder Duftstoffe auf der Haut und dem Haar in erhöhter Menge abzuscheiden. Gleichzeitig verbleiben die Parfümöle und Duftstoffe deutlich länger auf der Haut oder dem Haar haften. Dies führt zu einer erhöhten Akzeptanz derartiger Zusammensetzungen beim Verbraucher. Diese Ergebnisse sind besonders relevant für Zusammensetzungen wie Shampoos, Duschbädern, Kuren, Kurpackungen, Konditioniermitteln, leave-on Haarkuren, Stylingmitteln sowie das Haar fixierende und festigende Mittel.

Eine weitere Gruppe von ganz besonders bevorzugten Inhaltsstoffen der erfindungsgemäßen Zusammensetzungen sind Parfüms. Die hervorragenden und völlig überraschenden positiven Ergebnissen von Zusammensetzungen enthaltend die erfindungsgemäße Wirkstoffe und Parfüms, wurde bereits zuvor ausführlich beschrieben.

Mit dem Begriff Parfüm sind Parfümöle, Duftstoffe und Riechstoffe gemeint. Als Parfümöle seien genannt Gemische aus natürlichen und synthetischen Riechstoffen.

Beispiele für Riechstoffe, die in den erfindungsgemäßen Zusammensetzungen sein können, finden sich z. B. in S. Arctander, Perfume and Flavor Materials, Vol. I und II, Montclair, N. J., 1969, Selbstverlag oder K. Bauer, D. Garbe und H. Surburg, Common Fragrance and Flavor Materials, 3rd. Ed., Wiley-VCH, Weinheim 1997.

Der Verbraucher mag bei der Wahrnehmung der kosmetischen Zusammensetzungen, insbesondere hervorgerufen durch eine ästethisch ansprechende Verpackung, gegebenenfalls in Verbindung mit aromatischen Duftnoten, die erfindungsgemäße Zusammensetzung mit einem Genußmittel wie z.B. Süsswaren oder Getränken in Verbindung bringen. Durch diese Assoziation kann, insbesondere bei Kindern, eine orale Aufnahme bzw. ein Herunterschlucken der kosmetischen Zusammensetzung prinzipiell nicht ausgeschlossen werden. In einer bevorzugten Ausführungsform enthalten daher die erfindungsgemäßen Zusammensetzungen einen Bitterstoff, um ein Herunterschlucken bzw. eine akzidentielle Ingestion zu verhindern. Dabei sind erfindungsgemäß Bitterstoffe bevorzugt, die in Wasser bei 20 °C zu mindestens 5 g/l löslich sind.

Erfindungsgemäß hervorragend geeignet als Bitterstoffe sind quartäre Ammoniumverbindungen, die sowohl im Kation als auch im Anion eine aromatische Gruppe enthalten. Eine solche Verbindung ist das kommerziell z.B. unter den Warenzeichen Bitrex^{®} und Indige-stin^{®} erhältliche Benzyldiethyl((2,6-Xylylcarbamoyl)methyl)ammoniumbenzoat. Diese Verbindung ist auch unter der Bezeichnung Denatonium Benzoate bekannt.

Der Bitterstoff ist in den erfindungsgemäßen Zusammensetzungen in Mengen von 0,0005 bis 0,1 Gew.-%, bezogen auf den Formkörper, enthalten. Besonders bevorzugt sind Mengen von 0,001 bis 0,05 Gew.-%.

Vorteilhaft im Sinne der Erfindung können kurzkettige Carbonsäuren (N) als Inhaltsstoff in den Zusammensetzungen verwendet werden. Beispiele für besonders geeignete Hydroxycarbonsäuren sind Glycolsäure, Glycerinsäure, Milchsäure, Äpfelsäure, Weinsäure oder Citronensäure. Selbstverständlich umfasst die erfindungsgemäße Lehre auch, dass diese Säuren in Form von Mischsalzen beispielsweise mit Aminosäuren, verwendet werden. Dies kann erfindungsgemäß bevorzugt sein. Beispiele für Aminosäuren, welche als Mischsalze mit diesen Hydroxycarbonsäuren verwendet werden können, sind Carnitin, Taurin, Histidin, Lysin, Arginin und Ornithin. Ein typischer Vertreter der erfindungsgemäßen Mischsalze ist beispielsweise Carnitintartrat.

Selbstverständlich umfaßt die erfindungsgemäße Lehre alle isomeren Formen, wie cis - trans - Isomere, Diastereomere und chirale Isomere.

Erfindungsgemäß ist es auch möglich eine Mischung aus mehreren Wirkstoffen dieser Gruppe einzusetzen.

Erfindungsgemäß ganz besonders bevorzugt ist es als kurzkettige Carbonsäuren im Sinne der Erfindung die sogenannten Genusssäuren zu verwenden.

Diese erfindungsgemäßen Wirkstoffe sind in den Mitteln in Konzentrationen von 0,01 Gew.% bis zu 20 Gew.%, vorzugsweise von 0,05 Gew.% bis zu 15 Gew.% und ganz besonders bevorzugt in Mengen von 0,1 Gew.% bis zu 5 Gew.% enthalten.

Eine ganz besonders vielfältige und interessante kosmetische Wirkstoffgruppe sind Polyhydroxyverbindungen. Unter Polyhydroxyverbindungen im Sinne der Erfindung werden alle Substanzen verstanden, welche die Definition in Römpp's Lexikon der Chemie, Ausgabe von 1999, Verlag Georg Thieme, erfüllen. Demnach sind unter Polyhydroxyverbindungen organische Verbindungen mit mindestens zwei Hydroxygruppen zu verstehen.

Zusammenfassend sind erfindungsgemäße Zusammensetzungen bevorzugt, bei denen die Polyhydroxyverbindung ausgewählt ist aus Ethylenglycol, Propylenglycol, Polyethylenglycol, Polypropylenglycol, Glycerin, Glucose, Fructose, Pentaerythrit, Sorbit, Mannit, Xylit und ihren Mischungen.

Unabhängig vom Typ der eingesetzten Polyhydroxyverbindung mit mindestens 2 OH-Gruppen sind erfindungsgemäße Mittel bevorzugt, die, bezogen auf das Gewicht des Mittels, 0,01 bis 5 Gew.%, vorzugsweise 0,05 bis 4 Gew.%, besonders bevorzugt 0,05 bis 3,5 Gew.% und insbesondere 0,1 bis 2,5 Gew.% Polyhydroxyverbindung(en) enthalten.

Die erfindungsgemäßen Polyhydroxyverbindungen sind in den Zusammensetzungen in Konzentrationen von 0,01 Gew.% bis zu 20 Gew.%, vorzugsweise von 0,05 Gew.% bis zu 15 Gew.% und ganz besonders bevorzugt in Mengen von 0,1 Gew.% bis zu 10 Gew.% enthalten.

Weitere optionale Inhaltsstoffe, welche in kosmetischen Zusammensetzungen gemeinsam verwendet werden können, sind Konservierungsmittel. Als Konservierungsmittel finden die in Anlage 6, Teil A und B der europäischen Kosmetikverordnung aufgeführten Stoffklassen Verwendung. Besonders bevorzugt ist eine milde Konservierung, idealerweise ohne den Zusatz von typischen Konservierungsmitteln.

In einer weiteren besonders bevorzugten Art der erfindungsgemäßen Zusammensetzungen kann auch die Wasseraktivität in den erfindungsgemäßen Zusammensetzungen soweit reduziert werden, dass ein Wachstum von Mikroorganismen nicht mehr stattfinden kann. Hierzu werden insbesondere Glycerin und Sorbit verwendet.

Die erfindungsgemäßen Zusammensetzungen tragen mit dazu bei, dass die Konservierung in hervorragender Art und Weise mit den milden Konservierungszusätzen möglich ist. Aber auch der vollständige Verzicht auf Konservierungsmittel ist möglich und erfindungsgemäß bevorzugt.

Die Mengen an Konservierungsmittel betragen von 0 bis 5 Gew.%, bevorzugt von 0 - 2 Gew.%, besonders bevorzugt von 0 - 1 Gew.% und ganz besonders bevorzugt von 0 bis 0,8 Gew.% bezogen auf die Gesamtmenge der Zusammensetzung.

Der erfindungsgemäße Wirkstoffkomplex kann weiterhin in färbenden Mitteln verwendet werden. In diesen färbenden Mitteln sind selbstverständlich als weitere Inhaltsstoffe Farbstoffvorprodukte enthalten.

Weitere ganz besonders bevorzugte optionale Inhaltsstoffe der Zusammensetzungen sind Farbstoffvorprodukte. Unter Farbstoffvorprodukten sind Oxidationsfarbstoffvorprodukte vom Entwickler- (X1) und Kuppler-Typ (X2), natürliche und synthetische direktziehende Farbstoffe (Y) und Vorstufen naturanaloger Farbstoffe, wie Indol- und Indolin-Derivate, sowie Mischungen von Vertretern einer oder mehrerer dieser Gruppen

Als Farbstoffe zur Anfärbung der Zusammensetzungen können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

Der pH-Wert der erfindungsgemäßen Zubereitungen kann prinzipiell bei Werten von 2 - 11 liegen. Der pH - Wert wird je nach dem Zweck und der Verwendung der erfindungsgemäßen Zusammensetzung ganz gezielt ausgewählt und eingestellt. Für Färbemittel liegt er beispielsweise bevorzugt zwischen 5 und 11, wobei Werte von 6 bis 10 besonders bevorzugt sind. Für reinigende Zusammensetzungen liegt er beispielsweise zwischen 4 und 7,5, bevorzugt zwischen 4 und 6.

Zur Einstellung dieses pH-Wertes kann praktisch jede für kosmetische Zwecke verwendbare Säure oder Base verwendet werden. Bevorzugte Basen sind Ammoniak, Alkalihydroxide, Monoethanolamin, Triethanolamin sowie N,N,N',N`-Tetrakis-(2-hydroxypropyl)-ethylendiamin.

Üblicherweise werden als Säuren Genußsäuren verwendet. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt.

Auch protische Lösemittel, wie beispielsweise Wasser, und Alkohole können in den erfindungsgemäßen Zusammensetzungen enthalten sein. Als Alkohole finden alle physiologisch bedenkenlos verwendbaren Alkohole Verwendung, beispielsweise Methanol, Ethanol, Isopropanol, Propanol, Butanol, Isobutanol, Glykol, Glycerin und deren Mischungen untereinander. Der Anteil an protischen Lösemitteln ergänzt in jedem Fall die erfindungsgemäße Zusammensetzung auf 100 Gewichtsteile. Bevorzugt sind in den kosmetischen Zusammensetzungen mindestens 30 Gew.% protische Lösemittel, besonders bevorzugt mindestens 50 Gew.% und ganz besonders bevorzugt mindestens 75 Gew.% sowie höchst bevorzugt mindestens 85 Gew.% protische Lösemittel enthalten.

Weiterhin können in einer ganz besonders bevorzugten Ausführungsform der Erfindung die UV - Filter (I) verwendet werden. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Zwei bevorzugte UV-Filter mit kationischen Gruppen sind die als Handelsprodukte erhältlichen Verbindungen Zimtsäureamidopropyl-trimethylammoniumchlorid (Incroquat®UV-283) und Dodecyldimethylaminobenzamidopropyl-dimethylammoniumtosylat (Escalol^{®} HP 610).

Selbstverständlich umfaßt die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter (I) sind in den erfindungsgemäß verwendeten Mitteln üblicherweise in Mengen 0,1-5 Gew.-%, bezogen auf das gesamte Mittel, enthalten. Mengen von 0,4-2,5 Gew.-% sind bevorzugt.

Weitere Wirk-, Hilfs- und Zusatzstoffe sind beispielsweise
Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
haarkonditionierende Verbindungen wie Phospholipide, beispielsweise Sojalecithin, Ei-Lecitin und Kephaline,
Dimethylisosorbid und Cyclodextrine,
symmetrische und unsymmetrische, lineare und verzweigte Dialkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether und Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether,
faserstrukturverbessernde Wirkstoffe, insbesondere Mono-, Di- und Oligosaccharide, wie beispielsweise Glucose, Galactose, Fructose, Fruchtzucker und Lactose,
Phospholipide, beispielsweise Sojalecithin, Ei-Lecithin und Kephaline,
quaternierte Amine wie Methyl-1-alkylamidoethyl-2-alkylimidazolinium-methosulfat,
Antischuppenwirkstoffe wie Piroctone Olamine, Zink Omadine und Climbazol,
Wirkstoffe wie Allantoin und Bisabolol,
Cholesterin,

Komplexbildner wie EDTA, NTA, β-Alanindiessigsäure, Iminodibersnsteinsäure und derne Salze, Etidronic acid und deren Salze und Phosphonsäuren,
Quell- und Penetrationsstoffe wie primäre, sekundäre und tertiäre Phosphate,
Trübungsmittel wie Latex, Styrol/PVP- und Styrol/Acrylamid-Copolymere
Perlglanzmittel wie Ethylenglykolmono- und -distearat sowie PEG-3-distearat,
Pigmente,
Reduktionsmittel wie z. B. Thioglykolsäure und deren Derivate, Thiomilchsäure, Cysteamin, Thioäpfelsäure und α-Mercaptoethansulfonsäure,
Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. die Monographie von K. H. Schrader verwiesen.

Ein weiterer Gegenstand der vorliegenden Erfindung ist ein Verfahren zur Haarbehandlung, bei dem die erfindungsgemäße Zusammensetzung in das feuchte Haar eingearbeitet wird, und die Zusammensetzung nach einer Einwirkzeit von wenigen Sekunden bis zu 5 Minuten mit kaltem bis zu etwa 40 °C warmem Wasser wieder ausgespült wird, gegebenenfalls das Haar mit einem Handtuch getrocknet wird, dann eine weitere pflegende und konditionierende Zusammensetzung B in das Handtuch trockene bis nasse Haar eingearbeitet wird, wobei diese Zusammensetzung wenige Sekunden bis hin zu 30 Minuten auf dem Haar verbleibt, und anschließend ausgespült wird, das Haar wiederum mit einem Handtuch getrocknet wird, gegebenenfalls gefolgt von einem Föhnschritt, um das Haar vollständig zu trocknen, und letztlich eine frisierende und stylende Zusammensetzung C in das Haar gegeben wird und vorzugsweise nicht wieder ausgespült wird. Sowohl die Zusammensetzung B als pflegende und konditionierende Zusammensetzung als auch die Zusammensetzung C können selbstverständlich wiederum die erfindungsgemäße Zusammensetzung enthalten. Bevorzugt ist es, wenn mindestens die Zusammensetzung B ebenfalls die erfindungsgemäße Zusammensetzung enthält.

Das erfindungsgemäße Verfahren umfasst auch, daß der erste Schritt der Haarwäsche gegebenenfalls wiederholt werden kann.

### Beispiele und Wirkungsnachweise

Alle Mengenangaben sind, soweit nicht anders vermerkt, Gewichtsteile.

### 1. Wirkungsnachweis

Vorbehandlung:
Haarsträhnen mit einem Gewicht von etwa 2 g und 18 cm Länge der Firma Kerling mit dem Herstellercode 6/0 hellbraun wurde mit einem üblichen starken Blondiermittel zweimal ultrablondiert. Anschließend wurde mit einem im Handel erhältnlichen starken Wellmittel (Poly Lock) zweimal dauergewellt. Es wurden dabei die Gebrauchsanweisungen der jeweiligen Mittel befolgt. Nach dem letzten Ausspülen wurden die Haare mit einem Föhn an der Luft getrocknet und 24 h bei Raumtemperatur (23°C) und 50% rel. Luftfeuchte konditioniert.

Behandlung:
Die so vorbehandelten Haarsträhnen wurden mit einer auf einen pH - Wert von 5,0 eingestellten und 1,0 % Aktivsubstanz an Polymer enthaltenden Wirkstofflösung der reinen Polymere in einer ersten Versuchsreihe behandelt. Hierzu wurden die Haarsträhnen 2 Minuten in die jeweilige Lösung bei einer Temperatur von 23°C getaucht. Anschließend wurde unter fließendem Wasser 1 Minute ausgespült und das überschüssige Wasser mit einem Handtuch abgetrocknet. Die Handtuch getrockneten Strähnen werden mit einem Kamm gekämmt und die Leichtigkeit des Kämmens durch mindestens 5 geschulte Testpersonen beurteilt. Jede Testperson beurteilt jede Haarsträhne. Jeder Testperson liegt dabei ein eigener Satz der zu beurteilenden Haarsträhnen vor. Haarsträhne aus. Die Beurteilung erfolgt nach dem Schulnotensystem. Eine 1 bedeutet sehr gut, eine 5 bedeutet sehr schlecht. Aus den Noten der einzelnen Testpersonen wird das arithmetische Mittel gebildet.

In einer zweiten Versuchsreihe wurde den auf pH 5 eingestellten 1,0 % Aktivsubstanz enthaltenden Polymerlösungen jeweils 10 % Aktivsubstanz unterschiedlicher Tenside zugegeben und die Behandlung sowie die Beurteilung wie in der ersten Versuchsreihe durchgeführt.

Die Ergebnisse sind in Tabelle 1 wiedergegeben.

**Tabelle 1: Ergebnisse der Naßkämmbarkeit:**

| Lfd. N r.: | Tensid(e) | Luviquat^{®} Style | Luviquat^{®} Excellence | Luviquat^{®} FC 370 | Luviquat^{®} FC 550 | Luviquat^{®} Care | Polymer W^{®} 37194 |
|---|---|---|---|---|---|---|---|
| 1 | Ohne | 3 | 5 | 3 | 4,5 | 3,5 | 4,5 |
| 2 | Akyposoft^{®} 45 NV (10%AS) | 1,75 | 1,75 | 3 | 2 | 2 | 1,75 |
| 3 | Plantapon^{®} LGC Sorb (10%AS) | 1,75 | 2 | 3 | 2 | 2 | 1,5 |
| 4 | Akyposoft^{®} 45 NV (5% AS) / Plantapon LGC Sorb (5%AS) | 1,25 | 1,25 | 2,5 | 1,5 | 1,5 | 1,5 |
| 5 | Texapon^{®} NSO (10%AS) | 5 | 3 | 5 | 5 | 4 | 3 |
| 6 | Texapon^{®} NSO (5%AS) / Plantapon^{®} LGC Sorb (5%AS) | 3 | 2,5 | 4,5 | 4 | 3 | 2 |
| 7 | Plantacare^{®} 1200 (4%AS) | 3 | 3 | 3 | 3 | 3 | 3 |
| 8 | Texapon^{®} NSO (6%AS) / Plantacare 1200 (4%AS) | 3,5 | 3 | 5 | 4,5 | 3,5 | 3 |
| | Kationische Ladungsdichte | 3,0 | 6,1 | 2.0 | 3,3 | 1,0 | 3,9 |
| | Molmasse g/u | 400.000 | 40.000 | 100.000 | 80.000 | 1.000.000 | >1.000.000 |
| | Produkt aus Ladungsdichte und Molmasse | 1.200.000 | 244.000 | 200.000 | 264.000 | 1.000.000 | >3.900.000 |

Ein Vergleich der Ergebnisse der Versuchsreihe 1 zeigt, dass weder die Ladungsdichte, noch die Molmasse eine zuverlässige und wenigstens von der Tendenz her richtige Auswahl eines geeigneten Polymeren zur Erzielung einer besseren Konditionierung von keratinischen Fasern ermöglicht.

Die Versuchsreihe 4 zeigt eindeutig, dass die Wirkung der erfindungsgemäßen Zusammensetzungen aus einer Kombination milder anionischer Tenside und einem kationischen und/oder amphoteren Polymer erheblich besser sind als die Wirkung der wässrigen Lösungen der Polymere allein.

Darüber hinaus zeigt sich durch einen Vergleich der Versuchsreihe 4 mit den Versuchsreihen 5, 7 und 8, dass die Wirkung der erfindungsgemäßen Zusammensetzung aus einer Kombination milder anionischer Tenside mit einem kationischen und/oder amphoteren Polymer den im Stand der Technik üblichen Zusammensetzungen deutlich überlegen ist.

Die Ergebnisse der Versuchsreihe 4 im Vergleich mit den Ergebnissen der Versuchsreihe 2 belegen, dass eine Kombination aus mindestens zwei milden anionischen Tensiden mit einem kationischen und/oder amphoteren Polymer zu einer gesteigerten Wirksamkeit im Vergleich zu Zusammensetzungen mit nur einem milden anionischen Tensid führt.

Der Rohstoff Plantapon LGC Sorb enthält zu 40 % den Rohstoff Plantacare 1200. Aus dem Vergleich der Ergebnisse der Versuchsreihen 3 und 7 zeigt sich, dass die Wirkung nicht auf den Anteil des Plantacare 1200 zurückzuführen ist sondern vielmehr auf der Anwesenheit des anionischen, carboxilierten Alkylpolyglucosides beruht.

Ein Vergleich der Versuchsreihen 5 und 6 zeigt, dass die Zusammensetzungen aus einem milden anionischen Tensid und einem kationischen und/oder amphoteren Polymer die konditionierenden Eigenschaften konventioneller Systeme deutlich verbessern.

Ein Vergleich der Versuchsreihen 6 und 8 zeigt auch hier wieder deutlich, dass der Effekt auf der Anwesenheit der Zusammensetzungen beruht, da ein Austausch des milden anionischen, carboxilierten Alkylpolyglucosides gegen ein nichtionisches Alkylpolyglucosid zu einem deutlich schlechteren Effekt führt.

Weiterhin zeigt sich, dass das Produkt aus der Ladungsdichte und der Molmasse für die Auswahl eines geeigneten Polymeren für die erfindungsgemäßen Zusammensetzungen ein geeignetes und zuverlässiges Hilfsmittel ist.

In einer zweiten Versuchsanordnung wurde die erfindungsgemäße Zusammensetzung in einer Färbecreme eingearbeitet. Nach der Ausfärbung wurde dann die Naßkämmbarkeit bestimmt. Hierzu wurde folgendes Testprotokoll gewählt. Die jeweiligen Polymere wurden zu 1,0 % Aktivsubstanz in die Färbecreme gemäß Tabelle 2 eingearbeitet. Als Haarsträhnen wurden nicht vorbehandelte Haarsträhnen des Typs Kerling hellbraun, Code 6/0 mit einer Länge von 18 cm und einem Gewicht von ca. 2 g verwendet. Als Entwickler für die Färbecreme wurde die Rezeptur gemäß Tabelle 3 verwendet. Zur Färbung wurden jeweils 4,0 g der Färbecreme und des Entwicklers miteinander vermischt und somit insgesamt 8 g der Färbezusammensetzung auf jeweils eine Haarsträhne von 2,0 g aufgetragen. Die Ausfärbund erfolgte bei 32 °C und wurde über 30 Minuten durchgeführt. Nach dieser Zeit wurde 2 Minuten unter fließendem Wasser von 23 °C abgespült. Die Haarsträhnen wurden mit dem Handtuch getrocknet und anschließend wie bereits zuvor beschrieben von erfahrenen Testpersonen naß gekämmt. Als Vergleich diente eine Zusammensetzung, welche kein Polymer enthielt. Die Ergebnisse sind in Tabelle 4 zusammengefaßt.

**Tabelle 2: Färbecreme:**

| Lfd. Nr.: | Rohstoff | Menge in Gew.% |
|---|---|---|
| | Stenol^{®} 1618 | 9,0 |
| | Lorol^{®} C12-18 | 3,0 |
| | Texapon^{®} NSO | 7,0 |
| | Eumulgin^{®} B1 | 0,5 |
| | Eumulgin^{®} B2 | 0,5 |
| | Isostearinsäure | 2,0 |
| | Edenor^{®} C14 | 0,5 |
| | Kaliumhydroxid (50 %ig) | 1,2 |
| | Natriumsulfit | 0,5 |
| | Ascorbinsäure | 0,4 |
| | Ammoniumsulfat | 0,5 |
| | Turpinal^{®} SL | 0,2 |
| | Parfüm | 0,4 |
| | p-Toluylendiaminsulfat | 0,36 |
| | 3-Methyl-4-aminophenol | 0,03 |
| | 3-Aminophenol | 0,02 |
| | Resorcin | 0,13 |
| | 2-Methylresorcin | 0,03 |
| | 2-Methylamino-3-amino-6-methoxypyridin | 0,015 |
| | 2,7-Dihydroxynaphthalin | 0,1 |
| | Wasser | Ad 100 |
| | Plantapon^{®} LGC Sorb | 5,0 |
| 1 | Ohne Polymer und ohne Plantapon^{®} LGC Sorb | ----- |
| 2 | Ohne Polymer | ----- |
| 3 | Luviquat^{®} Style | 1,0 (Aktivsubstanz) |
| 4 | Luviquat^{®} Excellence | 1,0 (Aktivsubstanz) |
| 5 | Luviquat^{®} FC 550 | 1,0 (Aktivsubstanz) |
| 6 | Luviquat^{®} Care | 1,0 (Aktivsubstanz) |
| 7 | Polymer W^{®} 37194 | 1,0 (Aktivsubstanz) |
| | pH - Wert mit Ammoniaklösung (25%) | Eingestellt auf 9,5 bis 10,0 |

**Tabelle 3: Zusammensetzung des Entwicklers:**

| Rohstoff | Gew.% |
|---|---|
| Wasserstoffperoxid (50%ig) | 12,0 |
| Turpina^{@}l SL | 1,5 |
| Texapon^{®} NSO | 2,0 |
| Acrysol^{®} 33 | 12,0 |
| Wasser | Ad 100 |
| pH - Wert mit Ammoniaklösung (25%ig) | Auf pH 3,5 - 4,0 eingestellt |

**Tabelle 4: Ergebnisse der Naßkämmbarkeit**

| Lfd.Nr.: | Ergebnis Naßkämmbarkeit |
|---|---|
| 1 | 3,5 |
| 2 | 3,0 |
| 3 | 1,3 |
| 4 | 2,2 |
| 5 | 2,7 |
| 6 | 1,7 |
| 7 | 1,6 |

Aus diesen Veraleichsversuchen ergibt sich eindeutig, dass eine Verbesserung der Konditionierung der gefärbten keratinischen Fasern eindeutig durch die Zusammensetzung erfolgt.
- Stenol^{®}: C₁₆-C₁₈-Fettalkohol (INCI-Bezeichnung: Cetearyl alcohol) (Cognis Deutschland)
- Lorol^{®}: C₁₂-C₁₈-Fettalkohol (INCI-Bezeichnung: Coconut Alcohol) (Cognis Deutschland)
- Texapon^{®} NSO: Natriumlaurylethersulfat ( 27 % Aktivsubstanz INCI: Sodium Laureth Sulfate) (Cognis Deutschland)
- Plantapon^{®} LGC: Alkylpolyglucosid-Carboxylat Natriumsalz; 30 % Aktivsubstanz (erfindungsgemäß) (Cognis Deutschland)
- Eumulgin^{®} B1: Cetylstearylakohol mit 12 EO-Einheiten (INCI-Bezeichnung: Ceteareth-12) (Cognis Deutschland)
- Eumulgin^{®} B2: Cetylstearylalkohol mit ca. 20 EO-Einheiten (INCI-Bezeichnung: Ceteareth-20)
- Turpinal^{®} SL: 1-Hydroxyethan-1,1-diphosphonsäure (INCI-Bezeichnung: Etidronic Acid, Aqua (Water)) (Solutia)
- Acrysol^{®} 33: 30 Gew.-% Aktivsubstanz in Wasser (INCI-Bezeichnung: Acrylates Copolymer) (Rohm & Haas)
- Edenor^{®} C14: Myristinsäure (INCI-Bezeichnung: Myristic acid)
- Plantacare^{®} 1200: C12-C16 Fettalkoholglucosid, 50 Gew.% in Wasser (INCI-Bezeichnung: Lauryl Glucoside)

## Patentansprüche

1. Kosmetische Zusammensetzung zur Konditionierung keratinischer Fasern und der Haut enthaltend mindestens ein mildes anionisches Tensid mit einem HET - CAM - Wert kleiner 1,5, **dadurch gekennzeichnet, dass** als milde anionische Tenside
a) eine Kombination aus a1) mindestens einer Ethercarbonsäure und a2) mindestens einem carboxilierten Alkylpolyglucosid verwendet wird und
b) kationischen und/oder amphoteren Polymeren.

2. Kosmetische Zusammensetzung nach Anspruch 1, **dadurch gekennzeichnet, daß** die beiden milden anionischen Tenside in einem Verhältnis von 10 : 1 bis 1 : 10 verwendet werden.

3. Kosmetische Zusammensetzung nach Anspruch 1, **dadurch gekennzeichnet,**
**daß** die kationischen und/oder amphoteren Polymere eine kationische Ladungsdichte von mindestens 1,0 meq/g aufweisen.

4. Kosmetische Zusammensetzung nach Anspruch 1, **dadurch gekennzeichnet, daß** das Produkt aus kationischer Ladungsdichte und Molmasse mindestens 100.000 ist.

5. Kosmetische Zusammensetzung nach einem der Ansprüche 1 bis 4, **dadurch gekennzeichnet, daß** das kationische und/oder amphotere Polymer ausgewählt ist aus den Produkten mit den Handelsbezeichnungen Luviquat Style, Luviquat Excellence, Luviquat FC 370, Luviquat FC 550, Luviquat Care, Polymer W 37194, den Merquat - Typen, den Mirapol - Typen, Polyethyleniminen, den Gafquat -Typen, den Jaguar - Typen, den Hexamethidrinen, den Polymeren aus Piperazinyleinheiten, den wasserlöslichen Poyaminoamiden, wie z.B. den Cartaretinen oder den Polyalkylenpolyaminen wie z.B. Hercosett- oder Delsetttypen.

6. Kosmetische Zusammensetzung nach einem der Ansprüche 1 bis 5, **dadurch gekennzeichnet, daß** weiterhin ein Ölkörper enthalten ist.

7. Kosmetische Zusammensetzung nach Anspruch 6, **dadurch gekennzeichnet, daß** der Ölkörper ausgewählt ist aus mindestens einer der Verbindungen ausgewählt aus den Silikonen, den Esterölen, den cyclischen organischen Carbonaten, den C6 bis C24 Dialkylethern oder den C6 bis C24 Monoglyceriden.

## Claims

1. A cosmetic composition for conditioning keratinic fibres and the skin, containing at least one mild anionic surfactant having a HET-CAM value of less than 1.5, **characterized in that**
a) a combination of a1) at least one ether carboxylic acid and a2) at least one carboxylated alkyl polyglycoside is used as the mild anionic surfactant, and
b) cationic and/or amphoteric polymers.

2. A cosmetic composition according to claim 1, **characterized in that** the two mild anionic surfactants are used in a ratio of from 10 : 1 to 1 : 10.

3. A cosmetic composition according to claim 1, **characterized in that** the cationic and/or amphoteric polymers have a cationic charge density of at least 1.0 meq/g.

4. A cosmetic composition according to claim 1, **characterized in that** the product of cationic charge density and molar mass is at least 100,000.

5. A cosmetic composition according to one of claims 1 to 4, **characterized in that** the cationic and/or amphoteric polymer is selected from the products having the trade names Luviquat Style, Luviquat Excellence, Luviquat FC 370, Luviquat FC 550, Luviquat Care, Polymer W 37194, the Merquat types, the Mirapol types, polyethylenimines, the Gafquat types, the Jaguar types, the hexamethidrines, the polymers from piperazinyl units, the water-soluble polyaminoamides such as the cartaretines, or the polyalkylene polyamines such as Hercosett types or Delsett types.

6. A cosmetic composition according to one of claims 1 to 5, **characterized in that** an oleosome is contained in addition.

7. A cosmetic composition according to claim 6, **characterized in that** the oleosome is selected from at least one of the compounds selected from the silicones, the ester oils, the cyclic organic carbonates, the C6 to C24 dialkyl ethers or the C6 to C24 monoglycerides.

## Revendications

1. Composition cosmétique pour le conditionnement de fibres de kératine et de la peau contenant au moins un agent tensio-actif anionique doux ayant une valeur HET-CAM inférieure à 1,5, **caractérisée en ce que** les tensioactifs anioniques doux utilisés sont
a) une combinaison de a1) au moins un acide éther carboxylique et a2) au moins un alkylpolyglucoside carboxylé et
b) des polymères cationiques et/ou amphotères.

2. Composition cosmétique selon la revendication 1, **caractérisée en ce que** les deux tensio-actifs anioniques doux sont utilisés dans un rapport de 10:1 à 1:10.

3. Composition cosmétique selon la revendication 1, **caractérisée en ce que** les polymères cationiques et/ou amphotères ont une densité de charge cationique d'au moins 1,0 méq/g.

4. Composition cosmétique selon la revendication 1, **caractérisée en ce que** le produit de la densité de charge cationique et de la masse moléculaire est d'au moins 100.000.

5. Composition cosmétique selon l'une des revendications 1 à 4, **caractérisée en ce que** le polymère cationique et/ou amphotère est choisi parmi les produits ayant les noms commerciaux Luviquat style, Luviquat Excellence, Luviquat FC 370, Luviquat FC 550, Luviquat Care, Polymer W 37194, les types Merquat, les types Mirapol, les polyéthylèneimines, les types Gafquat, les types Jaguar, les hexaméthidrines, les polymères d'unités pipérazinyle, les poyaminoamides hydrosolubles tels que les cartarétines ou les polyalkylènepolyamines telles que les types Hercosett ou Delsett.

6. Composition cosmétique selon l'une des revendications 1 à 5, **caractérisée en ce qu'**elle contient en outre un composant huileux.

7. Composition cosmétique selon la revendication 6, **caractérisée en ce que** le composant huileux est choisi parmi au moins un des composés choisis parmi les silicones, les huiles d'esters, les carbonates organiques cycliques, les dialkyléthers en C6 à C24 ou les monoglycérides en C6 à C24.
